# EUROPÄISCHE PATENTSCHRIFT

(11) **EP 2 879 662 B1**
(45) Veröffentlichungstag und Bekanntmachung des Hinweises auf die Patenterteilung: **08.02.2017**
(21) Anmeldenummer: 13744529.2
(22) Anmeldetag: 31.07.2013
(51) Int. Cl.: A61K 31/11, A61K 31/05, A61K 31/045, A61K 31/122, A61K 31/351, A61K 31/085, A61K 31/618, A23K 20/111

(54) **ZUSAMMENSETZUNGEN ZUR LINDERUNG VON GASTROINTESTINALTRAKTSTÖRUNGEN ODER DAMIT ASSOZIIERTER SYSTEMISCHER STÖRUNGEN VON WIEDERKÄUERN UND CAMELIDEN**
COMPOSITION FOR ALLEVIATING GASTROINTESTINAL DEFECTS OR SYSTEMIC SYMPTOMES ASSOCIATED HEREWITH IN RUMINANTS AND CAMELIDS
COMPOSITIONS POUR AMELIORER MALADIES GASTROINTESTINALES OU SYMPTOMES SYSTEMIQUES ASSOCIEES EN RUMINANTES ET CAMELIDES

(30) Priorität: 31.07.2012 DE 102012015029
(43) Veröffentlichungstag der Anmeldung: 10.06.2015
(73) Patentinhaber: Freie Universität Berlin, 14195 Berlin (DE)
(72) Erfinder: STUMPFF, Friederike, 12209 Berlin (DE); ROSENDAHL, Julia, 10719 Berlin (DE); ASCHENBACH, Jörg, 04288 Leipzig (DE)
(74) Vertreter: Maikowski & Ninnemann Patentanwälte Partnerschaft mbB
(86) Internationale Anmeldenummer: PCT/EP2013/066094
(87) Internationale Veröffentlichungsnummer: WO 2014/020067

(56) Entgegenhaltungen:
- EP-A1- 2 368 440
- EP-A2- 1 609 372
- WO-A2-2011/153299

## Beschreibung

### Technisches Gebiet

Die vorliegende Erfindung betrifft Futtermittelzusätze zur Linderung von Gastrointestinaltraktstörungen und damit in Zusammenhang stehender systemischer Störungen in einem Tier aus der Unterordnung der Wiederkäuer (Ruminantia) oder der (wiederkäuenden) Schwielensohler (Tylopoda). Ferner betrifft die vorliegende Erfindung Medikamente zur Behandlung von Krankheiten, die mit Störungen des Gastrointestinaltraktes assoziiert sind, insbesondere Pansenazidose und/oder Gebärparese.

### Hintergrund der Erfindung

In der heutigen Nutztierhaltung treten gastrointestinale Stoffwechselstörungen gehäuft auf, da ihre Fütterung aufgrund der Anforderungen der modernen Tierhaltung verändert werden musste, um eine ausreichende Erhaltung sowie Wachstum der Tiermasse für die Fleischproduktion und daneben eine ausreichende Produktion von Erzeugnissen wie Milch zu gewährleisten. Als problematisch erweist sich dabei unter anderem, dass die Resorptionsleistung der gastrointestinalen Organe nicht Schritt hält mit dem Bedarf.

Die normale Ration eines Wiederkäuers ist Grünfutter in Form von Gräsern und zellulolytischen Nebenprodukten aus landwirtschaftlicher Erzeugung. Diese werden entweder frisch als Weidegras oder Grünschnitt, in einer trockenen Form als Heu oder in einem konservierten Zustand als Silage verfüttert. Die Fähigkeit zur Verwendung dieser Materialien als Nahrungsmittelquellen ist nur möglich aufgrund einer prägastrischen bakteriellen Fermentation im Pansen, dem nicht fundusartigen Abschnitt des Tiermagens. Hier reduziert eine bakterielle Wirkung die Kohlenhydrate mit komplexer Struktur, Cellulose, Hemicellulose und Lignin und die assoziierten Nichtstruktur-Kohlenhydrate; Pektin, Stärken und Zucker zu kurzkettigen Fettsäuren, welche über die Pansenwand resorbiert werden und in den Kohlehydratstoffwechsel eingehen. Neben den Fettsäuren werden auch Stickstoffderivate wie Ammoniak und Elektrolyte wie Natrium, Calcium und Magnesium über die Pansenwand resorbiert und ins Blut aufgenommen.

Die im Pansen wachsenden Mikroorganismen werden nach dem Efflux aus dem Pansen einer Verdauungswirkung im fundusartigen Magen und Dünndarm unterzogen und stellen die wesentliche Proteinquelle des Wiederkäuers dar. Im Futter enthaltene Proteine werden hingegen von der mikrobiellen Flora des Pansens überwiegend zu Ammoniak abgebaut, welches wieder in die mikrobielle Proteinbiosynthese eingehen muss, um vom Tier verwertet zu werden.

Die Adaption der Wiederkäuer auf eine prägastrische Verdauung hat ein System des Zurückhaltens der aufgenommenen Ration entwickelt, was einen bedeutenden Teil des Mechanismus zur maximalen Extraktion der Energie aus schwerverdaulichen strukturellen Kohlehydraten darstellt. Dieses Zurückhalten erfordert einen gewissen Verzicht bei der Nahrungsaufnahme, welches weiter eingeschränkt wird bei Nahrungsmitteln, die auf Grünfutter basieren, da die gröbere aufgenommene Nahrung länger zurückgehalten werden muss, um eine wirksame Extraktion an Energie zu erzielen. Dies stellt ein spezielles Problem beim modernen domestizierten Wiederkäuern dar, da die Nährstoffnachfrage, die durch eine genetische Selektion hinsichtlich eines schnellen mageren Muskelwachstums oder hoher Niveaus der Milcherzeugung erzeugt wird, in großem Maße die Zufuhr übersteigen, die gebildet wird durch eine Fermentation der auf Grünfutter basierenden Ration.

Die Ration, die verfüttert werden muss, erfordert den Zusatz von großen Mengen an Nichtstruktur-Kohlehydraten (Stärken und Zucker), die in der Form von Körnern verfüttert werden. Weiterhin ist eine Zufütterung proteinreicher Nährstoffe (z.B. Sojaschrot oder Leguminosen) erforderlich, um den Bedarf an Proteinen zu decken. Diese Zusatzstoffe führen zu einer beschleunigten Fermentationstätigkeit im Pansen, wobei hohe Konzentrationen an kurzkettigen Fettsäuren und Ammoniak frei werden, die oft eine Quelle an physiologischem und metabolischem Stress darstellen. Insbesondere kommt es dabei häufig zu einer azidotischen Entgleisung des Pansenmilieus (Pansenazidose). Als eine Folge müssen Futterstrategien versuchen, eine Grünfutterverwendung zu maximieren ohne die für eine Aufrechterhaltung und Produktion notwendige Nährstoffzufuhr zu beeinträchtigen. Alternativ kann versucht werden, die Resorption der bei der Pansenfermentation entstehenden Fermentationsprodukte so zu optimieren, dass weniger oder keine gastrointestinalen Störungen, wie z.B. die Pansenazidose auftreten.

Empfohlene Behandlungen für Pansenazidose, als eine der häufigsten Gastrointestinalstörungen bzw. Erkrankungen schließen Verabreichung einer Mischung von Natriumbicarbonat, Formaldehyd, Magnesiumoxid und Kohle ein, um die sich rasch teilenden Bakterien zu töten (NebGuide G91-1047-A). Allerdings wird hierdurch auch der für die weitere Passage notwendige Abbau von Strukturfasern aus Grünfutter, Heu oder Silage durch bakterielle Enzyme gehemmt. Zur Neutralisierung der bei der Fermentation entstehenden Säuren werden weithin Puffer verwendet (Horn, 1979, Kennelly, 1999), die aber nicht wirksam genug zu sein scheinen, um die Tierindustrie zufrieden zu stellen. Die Schmackhaftigkeit der meisten Puffer ist gering und erfordert vorsichtige Handhabung, um reduzierte Futteraufnahme zu vermeiden. Ionophore Antibiotika, wie Monensin, Lasalocid und Salinomycin sind allgemein gegen Gram-positive Bakterien wirksam, einschließlich der hauptsächlichen ruminalen Lactat-bildenden Bakterien, S. bovis und Lactobacillus-Arten (Burrin und Britton, 1986, Coe, 1999, Nagaraja, 1985). Sie sind daher wirksam beim Verhüten akuter Azidose beim Übergang zu Hochkonzentrat-Fütterung, wenn Rinder zuerst den Mastviehlaufhofanlagen erreichen oder nach Kalben und stabilisieren den Pansen-pH. Ionophore verringern jedoch auch die Futteraufnahme. Es ist auch gezeigt worden, dass andere Antibiotikaklassen akute Azidose verhüten oder verbessern, einschließlich Virginamycin in Schafen (Thorniley et al., 1998) und des schwefelhaltigen Peptidantibiotikums Thiopeptin, das gegen *S. bovis* besonders wirksam ist (Armstrong, 1984). Jedoch wird anhaltende Verwendung von antibiotischen Futterzusatzstoffen u. A. wegen der Resistenzentwicklung mit Folgewirkungen für den Menschen nicht länger als ein geeignetes Management-Werkzeug angesehen (zum Überblick siehe: *The use of drugs in food animals: benefits and risks,* 1999).

Bei einer weiteren häufig auftretenden gastrointestinalen Störung bzw. Krankheit, der Gebärparese, sinkt der Blutcalciumspiegel auf Werte ab, die zu Störungen in der neuromuskulären Erregungsweiterleitung mit Lähmungsfolge führen. Ursache sind die hohen Verluste von Calcium mit der Milch, die insuffiziente Resorptionskapazität des Gastrointestinaltraktes, und die unzureichende Mobilisation aus den Körperspeichern (Knochen und Plasmaproteine). Hierzu trägt die alkalotische Stoffwechsellage des Wiederkäuers bei. Es werden als Therapie üblicherweise differenziertes Futter vor und nach dem Kalben verabreicht, wobei Ca-freies Mineralfutter in der Trockenstehzeit und Ca-reiches Mineralfutter ab der Geburt verabreicht werden. Alternativ werden hohe Gaben an Ca-Salzen und Vitamin D verabreicht. Allerdings können hohe Dosen an Vitamin D zu irreversiblen Verkalkungen des Weichteilgewebes führen (Littledike et al., 1986) und die differentielle Fütterung ist aufwändig durchzuführen. Weiterhin fördert Vitamin D den Einbau von Ca in den Knochen, woraus ein weiteres Absinken der Blutcalciumspiegel folgen kann. Bei der Verabreichung von hohen Konzentrationen von Calcium-Salzen kommt es zwar zu einer teilweisen Korrektur der alkalotischen Stoffwechsellage des Tieres mit anschließender Fähigkeit, vermehrt Calcium aus dem Knochen zu mobilisieren (Goff und Horst, 1993), nicht selten geht dieses jedoch einher mit einer erniedrigten Calciumaufnahme, wenn die Zufütterung zu lange dauert. Zu hohe Calcium-Dosen können hierbei sogar toxisch sein (Goff et al., 2002). Weiterhin kommen sog. "anionische" oder "saure" Salze wie z.B. Ammoniumsulfat zum Einsatz (Goff et al. 2004; Gelfert et al. 2010), mit denen die alkalotische Stoffwechsellage des Wiederkäuers korrigiert werden soll um die Mobilisation von Calcium aus Knochen und Plasmaproteinen zu fördern. Aber auch hier muss mit einer Minderung der Futteraufnahme gerechnet werden. Die Gebärparese stellt noch immer einen erheblichen, den Ertrag mindernde Komplikation dar und es besteht auch hier ein Bedarf an neuen Futterzusätzen oder Mitteln zur Verhütung und Linderung der Krankheit.

Aus der EP 1 609 372 A2 sind Futtermittelzusätze bekannt, die Thymol, Eugenol und/oder Zimtaldehyd enthalten und die zur Verbesserung der Milchproduktion von Kühen eingesetzt werden.

Die Aufgabe der vorliegenden Erfindung war es auf diesem Hintergrund, Futterzusätze bereitzustellen, die als Futterzusatz oder fester Bestandteil eines Futtermittels geeignet sind, eine optimale Verwertung von Futtermitteln in Tieren aus der Unterordnung der Wiederkäuer (Ruminantia) oder der (wiederkäuenden) Schwielensohler (Tylopoda) zu gewährleisten, indem sie der Entstehung von metabolisch bedingten subklinischen Störungen oder ausgeprägten Krankheiten, wie z.B. der Pansenazidose oder der Gebärparese vorbeugen, die Proteinverwertung steigern und/oder das Verhältnis der Futteraufnahme zur Leistung des Tieres (z.B. Milchproduktion) verbessern.

### Kurzdarstellung der Erfindung

Zur Lösung der Aufgabe stellt die vorliegende Erfindung Futterzusätze bereit, die Substanzen enthalten, die die Aktivität eines oder unterschiedlicher Transient Receptor Potential (TRP)-Kanäle modulieren können und somit als Agonisten und/oder Antagonisten dieser Kanäle klassifiziert werden können. Hierzu werden erfindungsgemäße Fütterungsmethoden vorgeschlagen, welche die Zufütterung oder pharmakologische Gabe von einem oder mehreren der im Anhang vermerkten TRP-Agonisten und/oder Antagonisten, oder von einen oder mehrere dieser TRP-Modulatoren enthaltenden Futterzusätzen in einer Konzentrationsspanne von 0,01 bis 10 g/kg Futter zur Beeinflussung der Resorptionsvorgänge in Wiederkäuern sowie in Cameliden umfassen.

Ohne an eine Theorie gebunden sein zu wollen wird aufgrund der in den Beispielen gezeigten Ergebnisse angenommen, dass TRP-Modulatoren die Geschwindigkeit, mit der Kationen wie Ammonium, Calcium, Magnesium und Protonen aus dem Pansen resorbiert werden, modulieren können. Als weiterer Vorteil beeinflussen die TRP-Modulatoren oder diese enthaltenden erfindungsgemäßen Futterzusätze die Resorption der aufgeführten Ionen aus dem Pansen derart, dass eine Verbesserung von Störungen des Gastrointestinaltraktes bewirkt wird. Weiterhin erfolgt durch die Veränderung des Resorptionsgeschehens im Gastrointestinaltrakt eine Verbesserung des Gesundheitszustandes und/oder der Leistung des Tieres, wobei z.B. vermehrter Einbau von Ammoniak in für das Tier verwertbares mikrobielles Protein bei verminderter Freisetzung in die Umwelt erfolgt. Vorzugsweise beeinflussen die erfindungsgemäßen Futterzusätze dabei nicht die Mikroflora im Darm der Wiederkäuer oder Schwielensohler.

Alternativ oder zusätzlich werden TRP-Modulatoren und sie enthaltende Zusammensetzungen bereitgestellt zur Verwendung in der Linderung einer Erkrankung eines Tieres im Gastrointestinaltrakt.

**Die Figuren zeigen**

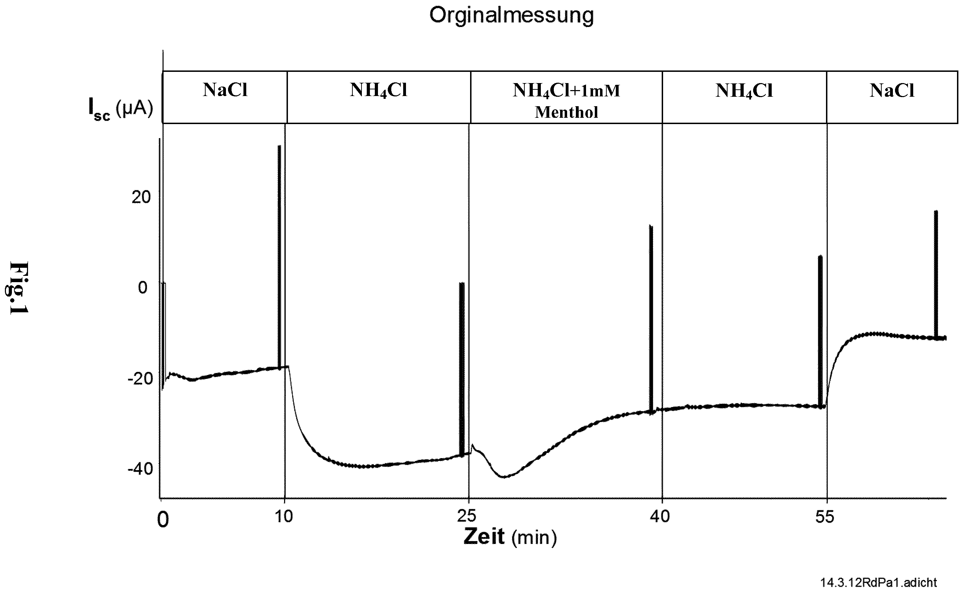

- **Fig. 1:**: Messung des transepithelialen Stroms über Pansenepithel vom Rind. Der Zusatz von 40mM NH₄CI zur mukosalen Lösung (Ringer) führt zu einer deutlichen Steigerung des Kurzschlussstromes (I_{sc}). Die Zusätzliche Gabe von Menthol (1mM) als TRP-Modulator führt zu einer weiteren Steigerung dieses Stroms, wobei von einer Öffnung von für NH₄⁺-Ionen durchlässigen Ionenkanälen ausgegangen wird.

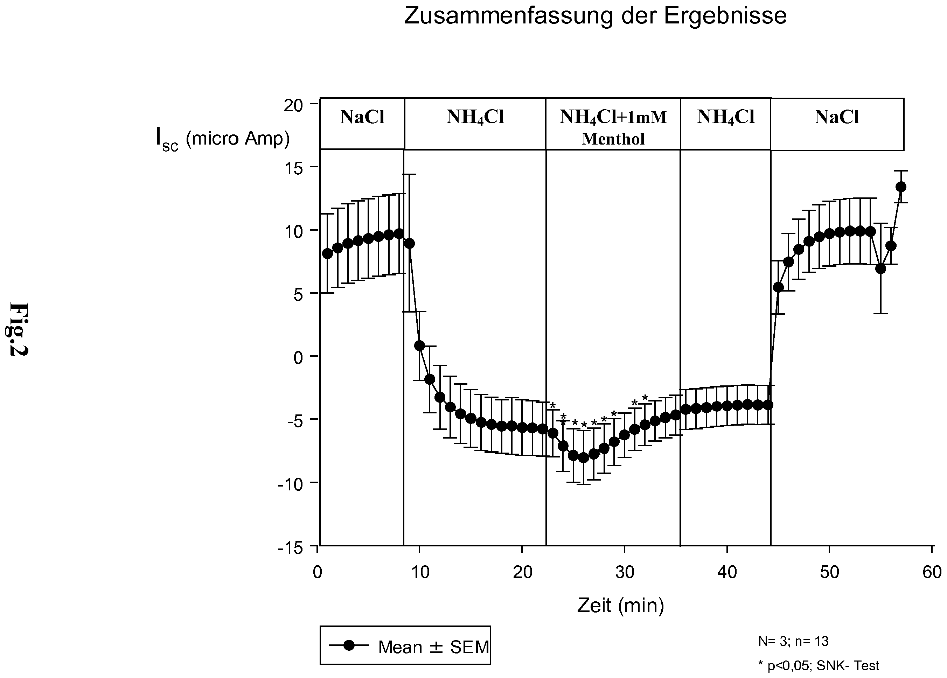

- **Fig. 2:**: Zusammenfassung der Ergebnisse. Aufgetragen wurden die Mittelwerte des transepithelialen Stroms (± SEM) über die Zeit von 13 Einzelmessungen an 3 verschiedenen Epithelien wie in Figur 1. Nach Gabe von Menthol (1 mmol·l⁻¹) als TRP-Modulator kommt es zu einer statistisch signifikanten Zunahme des Stromes über das Epithel (p<0,05; Student-Newman-Keuls). Ähnliche Ergebnisse wurden auch bei Versuchen mit Gabe einer geringeren Dosis (200 µmol·l⁻¹) Menthol) beobachtet.

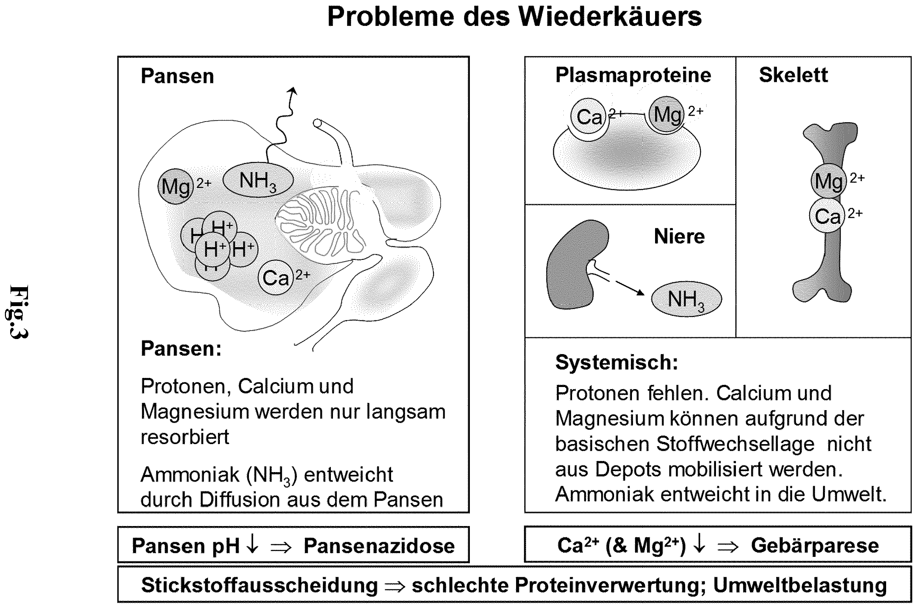

- **Fig.3:**: Probleme des Wiederkäuers. Die Resorptionskapzität des Pansens für Kationen wie Calcium, Magnesium und H⁺-Ionen (Protonen) ist begrenzt. Im Pansen kommt es zur azidotischen Entgleisung mit Reduktion der Proteinverwertung. Systemisch findet man hingegen einen Mangel an Protonen (= basische Stoffwechsellage oder Alkalose), welche die Mobilisation von Calcium und Magnesium aus den Depots in Knochen und Plasmaproteinen erschwert. Aus dem Pansen entweichendes Ammoniak (NH₃) verschärft die Pansenazidose und die systemische Alkalose. Außerdem wird die Umwandlung von Ammoniak in mikrobielles Protein für Milchbildung und Fleischansatz verhindert. Das systemisch aufgenommene Ammoniak ist giftig, muss ausgeschieden werden und führt zur Belastung der Umwelt.

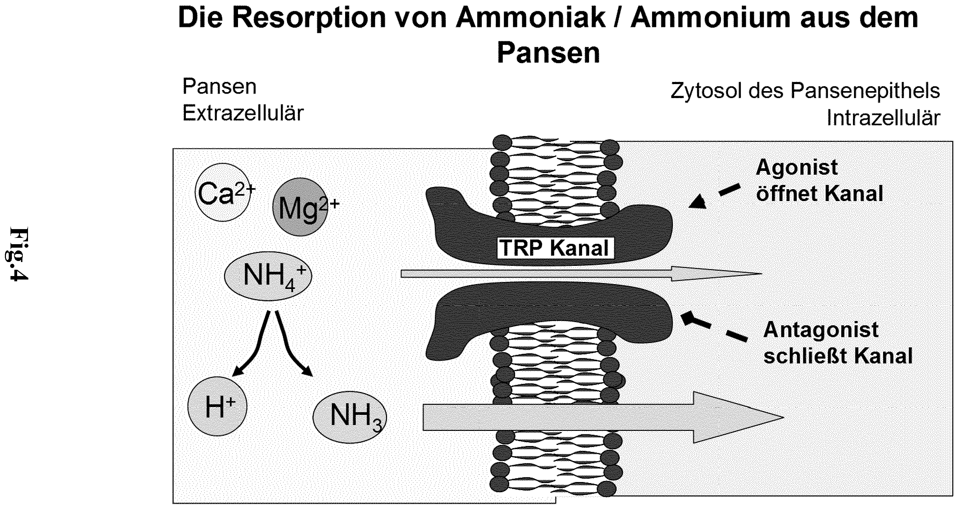

- **Fig.4:**: Die Resorption von Ammoniak / Ammonium aus dem Pansen. Das im Pansen befindliche Ammonium (NH₄⁺) spaltet sich fortlaufend zu Ammoniak (NH₃), wobei ein Proton (H⁺) abgespalten wird. Das NH₃ entweicht rasch aus dem Pansen, da die Lipidmembran der Zellen des Pansenepithels für lipidlösliche Substanzen wie Ammoniak (NH₃) hochpermeabel ist. Das Proton bleibt zurück und säuert den Pansen an, während das Eindringen von Ammoniak (NH₃) in die Zelle und anschließend in den Organismus Entstehung einer Alkalose (= basischer Stoffwechselentgleisung) beiträgt. Alternativ kann Ammonium (NH₄⁺) über die Zellmembran gelangen. Als Transportweg fungieren nicht selektive Kationenkanäle, welche zahlreiche Kationen über das Pansenepithel leiten, darunter auch Calcium und Magnesium (Martens and Harmeyer 1978; Lang and Martens 1999; Schweigel, Lang et al. 1999; Abdoun, Stumpff et al. 2005; Leonhard-Marek, Stumpff et al. 2005). Zahlreiche Agonisten und Antagonisten regulieren über verschiedene Mechanismen die Durchlässigkeit dieser Kanäle für Kationen und führen bei Exposition so zur Auslösung von sensorischen Empfindungen (Ramsey, Delling et al. 2006; Xu, Delling et al. 2006; Vriens, Nilius et al. 2008).

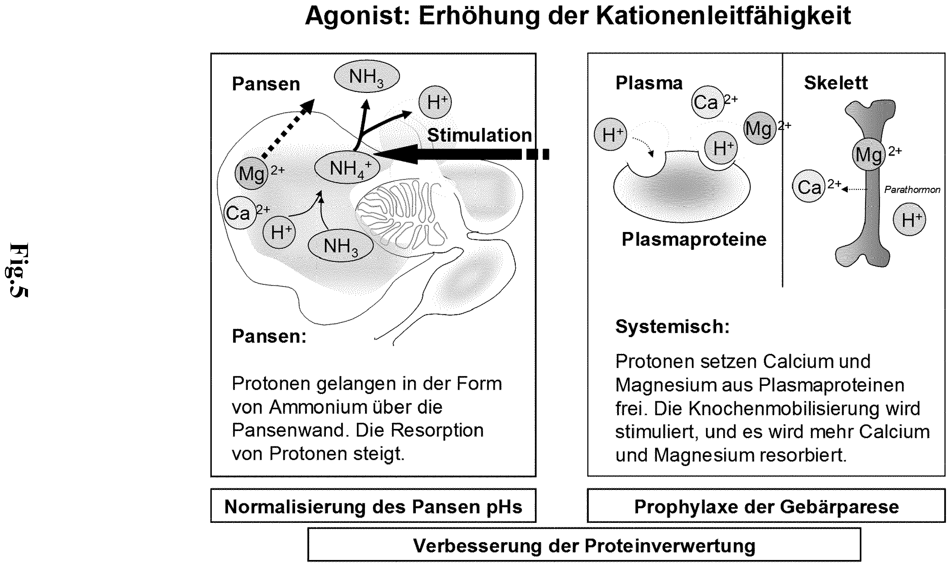

- **Fig. 5:**: Erhöhung der Kationenleitfähigkeit: Durch Gabe eines TRP-Agonisten wird die Resorptionskapazität des Pansens für Kationen gesteigert. Stickstoff, welcher von den Bakterien nicht verwertet werden kann, gelangt überwiegend in der protonierten Form als Ammonium (NH₄⁺) über die Pansenwand. Hierdurch werden Protonen aus dem Pansenlumen entfernt und der Pansen-pH normalisiert sich. Gleichzeitig wird die systemische Alkalose korrigiert. Protonen verdrängen Calcium und Magnesium aus der Bindung an Plasmaproteine und stimulieren die Sekretion von Parathormon für die Mobilisation aus dem Knochen. Eine verbesserte Resorptionskapazität des Pansenepithels für Kationen - darunter Calcium, Magnesium, und Protonen - unterstützt den Effekt. Der Plasmaspiegel von Calcium und Magnesium normalisiert sich und verhindert das Auftreten von Calciummangelzuständen wie der Gebärparese. Bei Normalisierung des pH-Wertes im Pansenlumen wird weiterhin ein verbessertes Bakterienwachstum mit vermehrter Nutzung des verbleibenden Ammoniaks für die Proteinbiosynthese erwartet.

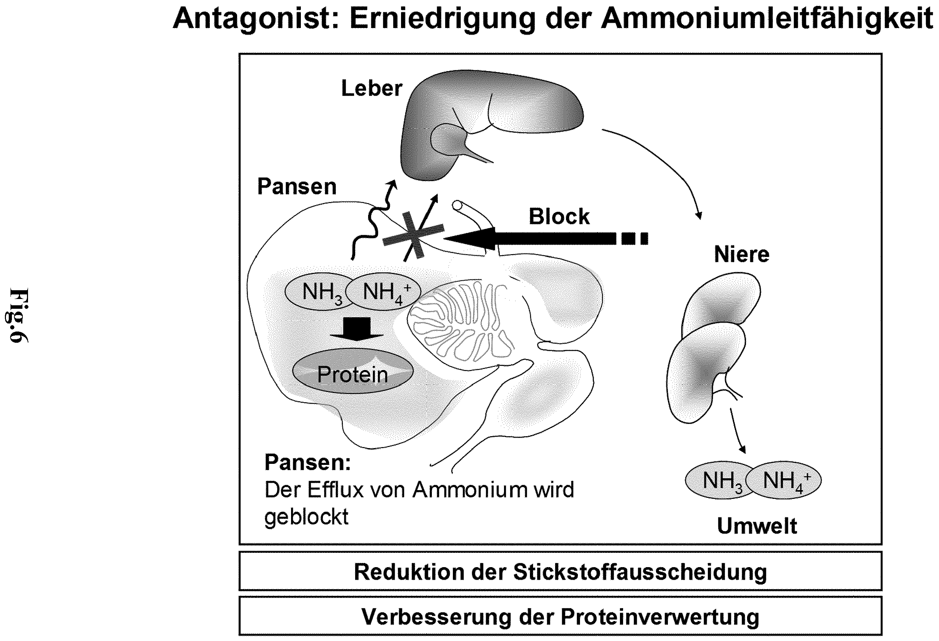

- **Fig. 6:**: Absenkung der Ammoniumleitfähigkeit: Durch Gabe eines TRP-Antagonisten, wird die Resorption von Ammonium (NH₄⁺) über die Pansenwand reduziert. Mehr Stickstoff bleibt im Pansen und kann in bakterielles Protein integriert werden. Die Zufütterung von Proteinen kann reduziert werden, die Leberbelastung sinkt und es gelangt weniger Stickstoff in die Umwelt. Da eine gleichzeitige Reduktion der Calcium- und Protonenaufnahme nicht ausgeschlossen werden kann, sollte diese Anwendungsform vor allem bei Fleischrindern in Betracht kommen, bei denen Gebärparese (=Hypocalcämie) nicht auftritt. Es erscheint aber auch denkbar, dass sich unter den zu schützenden Substanzen solche befinden, welche die Durchlässigkeit der Pore so modulieren, dass vermehrt zweiwertige Kationen wie Calcium und Magnesium aufgenommen werden bei Verdrängung der einwertigen Kationen (wie Ammonium) aus der Pore. Vermehrt auftretende Pansenazidosen werden als mögliche unerwünschte Nebenwirkung der Gabe eines TRP-Antagonisten erwartet. TRP-Antagonisten könnten z.B. bei Freilandhaltung mit hohem Rauhfutteranteil zum Einsatz kommen. Hier tritt die Pansenazidose seltener auf.

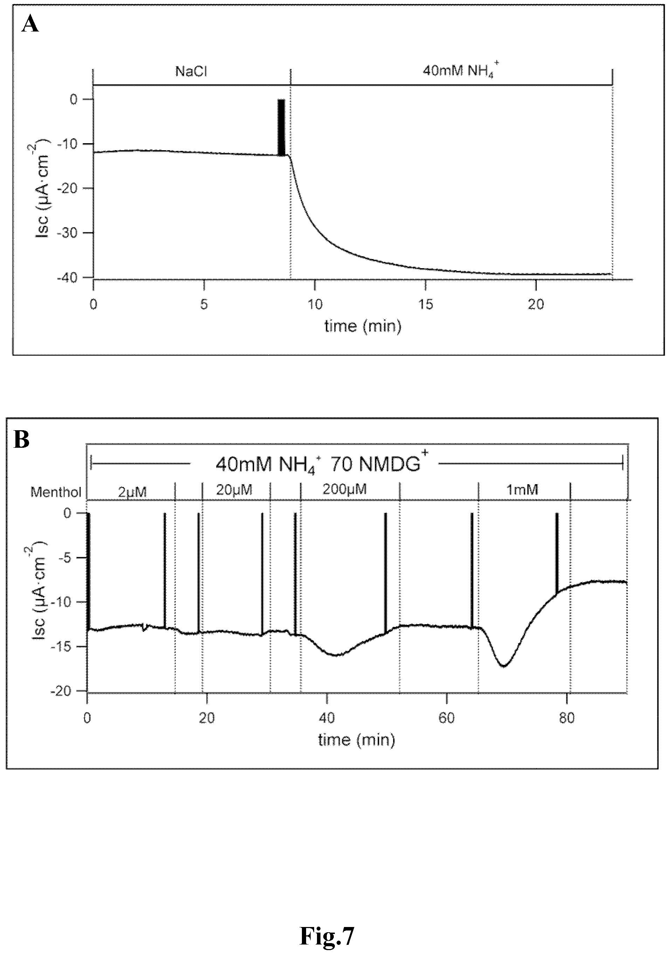

- **Fig. 7:**: Messungen in der horizontalen Ussingkammer. **A:** Der Ersatz von 40mM NMDG+ durch eine äquimolare Menge NH₄⁺ führt zu einer signifikanten Steigerung des transepithelialen Stroms. Die Messung belegt, dass das Epithel Ammonium in der ionisierten Form transportiert. **B:** Effekte von steigenden Konzentrationen von Menthol auf den transepithelialen Strom.

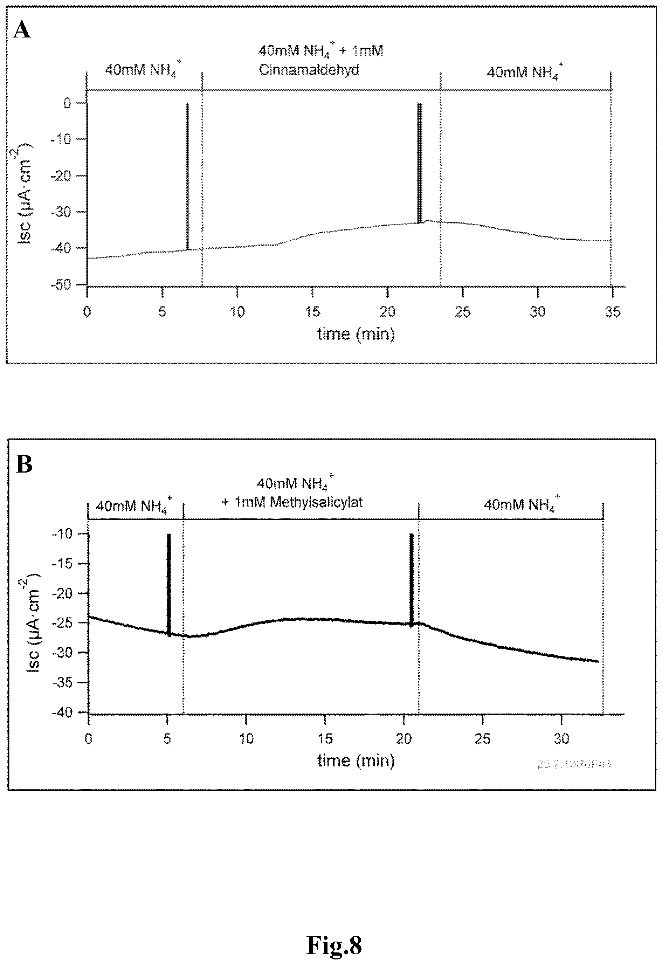

- **Fig. 8:**: Messungen in der horizontalen Ussingkammer. **A:** Effekt von Zimtaldehyd auf den transepithelialen Strom. **B:** Effekt von Methylsalicylat auf den transepithelialen Strom.

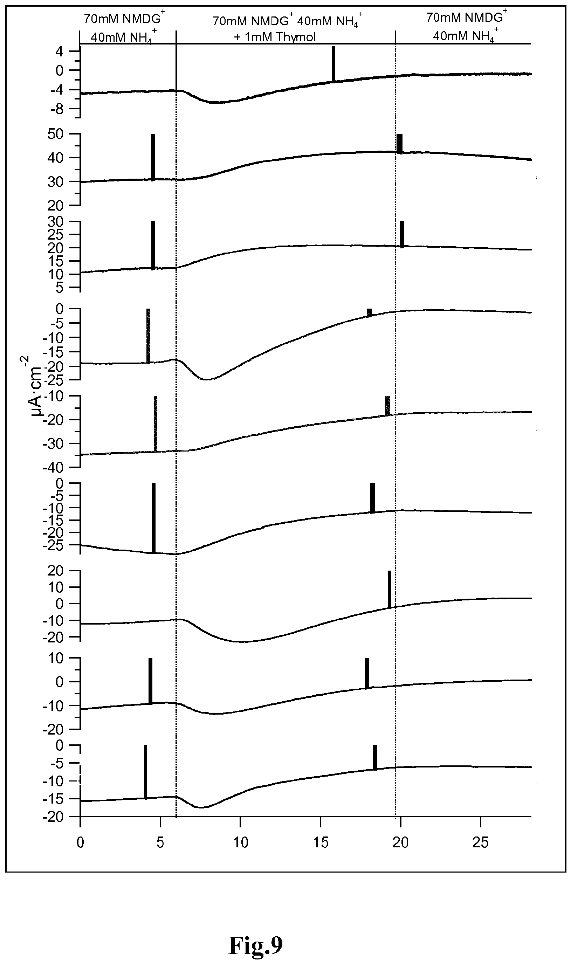

- **Fig. 9:**: Messungen in der horizontalen Ussingkammer. Vergleichende Darstellung von neun Messungen bei denen 1 mM Thymol in eine NH4+ hältige Na+-freie Perfusionslösung gegeben wurde.

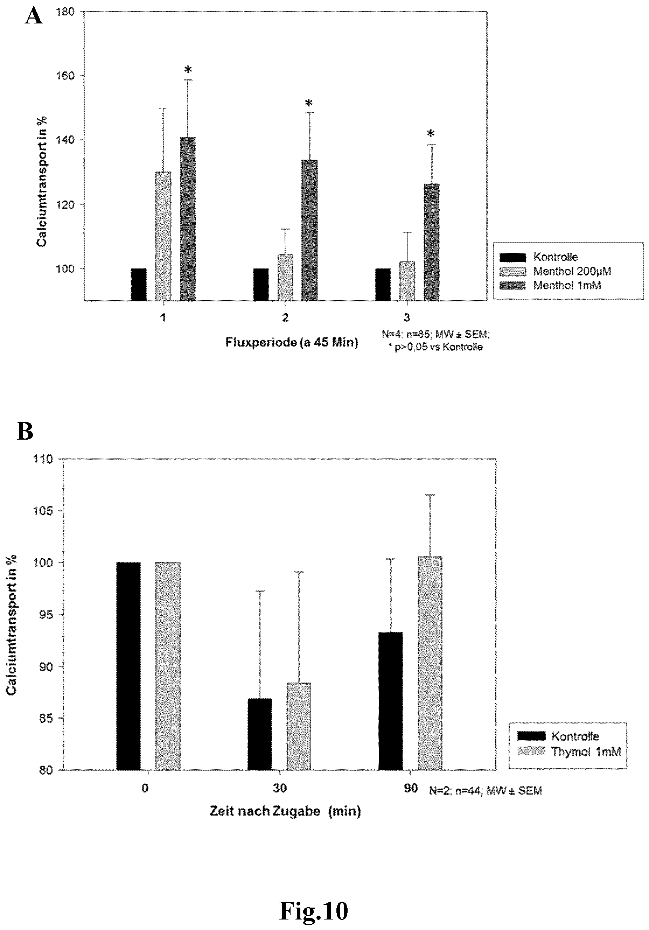

- **Fig. 10:**: Calciumflux über das Pansenepithel. **A:** Konzentrationsabhängige Steigerung des Calciumtransports über das Pansengewebe nach Zugabe von Menthol in zwei verschiedenen Konzentrationen im Vergleich zur Kontrollgruppe. N=4(Rinder) n=85 (Epithelien). **B:** Veränderung des Calciumtransports über das Pansengewebe nach Zugabe von Thymol im Vergleich zur Kontrollgruppe. N=2 (ein Rind, ein Schaf); n=44 (Epithelien).

### Definitionen und Begriffe

Soweit nachfolgend der Kontext nicht eindeutig etwas anderes ergibt, ist bei der Verwendung von Singular-Formen bzw. Plural-Formen stets sowohl die Mehr- als auch die Einzahl umfasst.

Der Begriff einer "Zusammensetzung" umfasst im Sinne der Erfindung Medikamente und Futterzusatzmittel und ist dadurch gekennzeichnet, dass zumindest einer der Bestandteile oder Inhaltsstoffe ein TRP-Kanal Modulator im Sinne der vorliegenden Erfindung ist. Beispiele von TRP-Modulatoren im Sinne der vorliegenden Erfindung sind in den Tabellen 10 bis 13 aufgelistet. Unter einem "TRP-Modulator" werden Substanzen verstanden, die einen oder mehrere TRP-Kanäle aktivieren ("TRP-Agonisten"), inhibieren ("TRP-Antagonisten") oder auch Substanzen, die auf unterschiedliche TRP-Kanäle eine unterschiedliche Wirkungen haben.

TRP-Kanal-Proteine bilden 6-Transmembrandomänen umfassende TRP-Kanäle, die Kationenfluss entsprechend derer elektrochemischen Gradienten vermitteln, wodurch die intrazellulären Ca2+ und Na+ Ionenkonzentrationen angehoben und die jeweilige Zelle depolarisiert wird. Auch andere Kationen können die Pore passieren, wobei die selektive Permeabilität vom Kanaltyp abhängt (Owsianik, Talavera et al. 2006). Nach der herrschenden Nomenklatur werden die Gene für die 28 bekannten Kanaluntereinheiten in 7 Familien unterteilt.

Viele der TRP-Kanäle können durch multiple Stimuli aktiviert werden. Diese polymodale Sensitivität legt nahe, dass die physiologisch relevanten Stimuli eines bestimmten TRP-Kanals abhängig von dem jeweiligen zellulären Kontext sind. Ferner führt die Kooperation zwischen unterschiedlichen TRP-Kanälen dazu, dass es bezüglich interagierender Substanzen nicht immer einfach ist, die Abgrenzung zwischen einem Agonisten und einem Modulator zu treffen.

Die TRP-Kanäle haben mannigfaltige Funktionen als Sensoren, die es einzelnen Zellen und dem jeweiligen Organismus erlauben Änderungen in der Umgebung festzustellen, umfassend Temperaturänderungen, Detektion von Berührungen, Schmerz, Geschmack und von bestimmten chemischen Substanzen. (siehe Ramsey et al., 2006; und Vriens et al., 2008) Aber auch eine Beteiligung dieser Kanäle an der epithelialen Resorption von Calcium (Lambers, Bindels et al. 2006) und Magnesium (Voets, Nilius et al. 2004) ist belegt.

Basierend auf den hier gezeigten Versuchsergebnissen ist unter Zugabe der TRP-Agonisten und/oder Antagonisten mit einer Veränderung (Steigerung beim Einsatz von Agonisten; Hemmung beim Einsatz von Antagonisten) der elektrophysiologischen Parameter und der Transportraten über das Pansenepithel zu rechnen.

Die für den Säuger relevante "TRP-Kanal-Familie" besteht aus sechs Subfamilien:
- TRPC (klassische)
- TRPV (Vanilloid-Rezeptor)
- TRPM (Melastatin)
- TRPA1 (ANKTM1)
- TRPP (Polycystin)
- TRPML (Mucolipin)

Diese Subfamilien lassen sich in Untergruppen unterteilen, von denen zahlreiche im Gastrointestinaltrakt des Säugetiers exprimiert werden.

Im Sinne der vorliegenden Erfindung werden unter TRP-Agonisten und/oder Antagonisten insbesondere entsprechende Agonisten und/oder Antagonisten der folgenden Kanäle verstanden:
TRPV1, TRPV2, TRPV3, TRPV4, TRPV5, TRPV6
TRPA1, TRPM3, TRPM5, TRPM6, TRPM7, TRPM8.
Aus den Begriffen und der Funktion der TRP-Kanäle als Ionenkanälen ergibt es sich, dass ein Agonist im Wesentlichen die Ionenleitfähigkeit des Pansenepitels über den jeweils beeinflussten Kanal steigert, während ein Antagonist diese senkt.

Der Begriff der "Wiederkäuer" oder "Ruminantia" umfasst im Sinne der Erfindung Tiere aus der Unterordnung der Paarhufer (Artiodactyla), die durch einen mehrteiligen Wiederkäuermagen charakterisiert sind der den Tieren u.a. eine effiziente Verdauung von Zellulose erlaubt. Der Magen zerfällt bei diesen Tieren in vier Abteilungen, die drei Vormägen, nämlich den Pansen (auch Wanst, Zottenmagen oder Rumen), den Netzmagen (auch Haube, Reticulum oder Ollula), den Blättermagen (auch Buchmagen, Psalter, Omasus oder Psalterium) und dem Hauptmagen, dem Labmagen (auch Abomasum oder Abomasus). Als zu den Wiederkäuern zugehörig werden infolgedessen Tieren aus den Familien der Giraffenartigen (Giraffidae), der Moschushirsche (Moschidae), der Gabelhornträger (Antilocapridae), der Hirsche (Cervidae) und der Hornträger (Bovidae) gezählt. Insbesondere werden im Sinne der vorliegenden Erfindung zu den Wiederkäuern Tiere aus der Gruppe umfassend: Rinder, Schafe, Ziegen, Giraffen, Yaks, Rothirsche, Damhirsche, Elche, Rehe, Antilopen, Büffel und Bisons gezählt.

Der Begriff der "(wiederkäuenden) Schwielensohler (Tylopoda)" umfasst im Sinne der Erfindung Tiere aus der Unterordnung der Paarhufer, deren Magenaufbau trotz fehlender Homologie dem der Wiederkäuer ähnelt. Er weist im Vergleich zu den Wiederkäuern einen reduzierten Blättermagen (Omasum) auf, der deswegen teilweise als dreigliedrig dargestellt wird. Als hierzu zugehörig werden Tiere aus der Familie der Kamele (Camelidae), der Gattungen der Altweltkamele (Camelus), Lamas (Lama) und Vikunjas (Vicugna) gezählt. Insbesondere werden im Sinne der vorliegenden Erfindung zu den Schwielensohler Tiere aus der Gruppe umfasend: Trampeltiere, Dromedare, Alpakas, Lamas, Guanakos und Vikunjas gezählt.

Der Begriff der "Pansenazidose" umfasst eine Stoffwechselstörung bei Wiederkäuern und bei Schwielensohlern, welche hauptsächlich infolge zu großer Mengen kohlenhydratreicher und zu geringer Mengen strukturreicher Futtermittel in der Ration auftritt (Dirksen et al., 2002). Die Pansenazidose ist durch eine Übersäuerung des Pansens, d. h. einen niedrigen pH-Wert des Pansensaftes, in der Regel unter pH 5,5, gekennzeichnet (der Normalwert liegt bei etwa pH 5,9 und darüber). Die Pansenazidose kann zu erheblichen Störungen in der Zusammensetzung der Pansenflora und in der Folge zur Schädigung der Pansenschleimhaut (Verätzung) führen.

Der pH des Pansensaftes kann nach dessen Entnahme gemessen werden. Eine Entnahme kann durch eine oral eingeführte Sonde stattfinden, der Aussagewert ist hierbei jedoch durch den stark alkalischen Speichel beeinträchtigt. Besser geeignet für die Pansensaftentnahme ist deswegen die Entnahme per Punktion des kaudoventralen Pansensackes (Ruminozentese) von ventrolateral, wie beschrieben bei Nordlund et al. 1994 und 1995. Alternativ kann der pH-Wert auch durch den Einsatz einer Sonde/Bolus (z.B. der Firma smaXtec animal care sales GmbH; Graz, AUSTRIA) über längere Zeit direkt im Magen aufgezeichnet und kabellos ausgelesen werden, wie beschrieben z.B. bei Gasteiner et al., (2009) im Abschnitt Material und Methode auf Seiten 3-5, dessen Offenbarungsgehalt durch Bezugnahme in die vorliegende Anmeldung eingeschlossen ist. Die Messung sollte etwa 2-4 Stunden nach der Fütterung immer bei mehreren Tieren einer Herde stattfinden, wobei die Diagnose einer Pansenazidose als gesichert gilt, wenn erniedrigter pH bei mindestens 25% der Tiere festgestellt wird.

Akute Pansenazidose ist durch eine steile Abnahme des pH-Werts mit einer hohen Konzentration ruminaler Milchsäure (50 -100 mM) gekennzeichnet. Die ruminale mikrobielle Population macht eine signifikante Verschiebung durch, wobei die Zahl Gram-positiver milchsäurebildender Bakterien stark zunimmt. Der fallende pH führt zum Tod Gramnegativer Bakterien und zur Verringerung oder vollständigem Verschwinden begeißelter Protozoen. Die subakute Pansenazidose (zwischen 5,2 und 5,8 pH) und die chronisch-latente metabolische Azidose sind durch eine verringerte Futteraufnahme (Inappetenz) und dadurch geringere körperliche Leistung, u. a. Milch- und Mastleistung, charakterisiert (Enemakr et al., 2004, Garret et al. 1999). Es kann auch zu Veränderungen der Kotkonsistenz führen, der graugrün gefärbt und pastös bis suppig sein kann. Bei mittelschwerer Ausprägung der Pansenazidose stellt der Wiederkäuer die Nahrungsaufnahme ein und der Milchfluss versiegt. Weitere Symptome sind schwere Verdauungsstörungen, wie Koliken und Durchfall, sowie Teilnahmlosigkeit, Schwanken und Lahmheit. Entsprechend dem Vorhandensein von Symptomen, oder deren Fehlen/schwacher Ausprägung kann die Pansenazidose als subklinisch oder klinisch eingestuft werden. Bei schweren Fällen erscheinen die Tiere träge und apathisch, liegen fest es kann auch Stöhnen und Zähneknirschen beobachtet werden. Bei störmischen Verlauf zeigen sich Unruhe, Muskelzittern, schmerzhaftes Anziehen der Gliedmaßen oder auch Kolikerscheinungen, Schweißausbruch, Niederwerfen und Wälzen sowie schwere Diarrhoe mit gelbgrünen, wässrig-schaumigen, nicht selten blutigen Ausscheidungen. Bei schwerem Verlauf, kommt es nach den vorher beschriebenen Symptomen zum Festliegen des Tieres die apathisch, später komatös sind und hohe Herzfrequenzen von > 120 Schlägen/min aufweisen. Ohne Behandlung kann es zum Verenden des Tieres kommen.

Infolge von sekundär entstehendem Thiaminmangel kann zusätzlich Hirnrindennekrose auftreten.

Eine häufige Ursache für Pansenazidose ist ein zu hoher Anteil leichtverdaulicher Inhaltstoffe des Futters (z. B. Stärke, Zucker), wie beispielsweise ein zu hoher Anteil an Konzentratfutter in Gesamtfutterrationen und ein zu niedriger Anteil strukturierter Rohfaser. Auch plötzliche Futterumstellungen können zu Azidose führen. Da die durch Abbau von Konzentratfutter gewonnenen Kohlenhydrate leicht verdaulich sind, entstehen beim mikrobiellen Abbau der Stärke anfangs große Mengen von kurzkettigen Fettsäuren. Zudem sinkt die Aktivität des Wiederkäuens, wodurch zu wenig alkalischer und mit Puffern angereichter Speichel in den Pansen gelangt und der Panseninhalt übersäuert. Dadurch wird die Zusammensetzung der Mikroflora im Pansen beeinträchtigt, es kommt zu einer Zunahme Milchsäure bildender Mikroorganismen und einer weiteren Absenkung des pH, so dass die Fermentation zu flüchtigen Fettsäuren, die über die Pansenwand resorbiert und vom Wiederkäuer energetisch verwertet werden können, abnimmt. Laktat akkumuliert hingegen im Pansen, der Pansen pH sinkt, und die Pansenwand wird geschädigt bis hin zur Auflösung der Barrierefunktion mit Bildung von Leberabszessen und anderen systemischen Manifestationen (Nocek 1997; Owens, Secrist et al. 1998; Kleen, Hooijer et al. 2003; Krause and Oetzel 2006).

Der Begriff "Gebärparese" (auch Hypokalzämie oder "Milchfieber") beschreibt eine Stoffwechselerkrankung von laktierenden Tieren wie Milchkühen. Dem Namen entsprechend ist die Erkrankung durch ein Absinken des Gesamtkalziumgehalts im Plasma unter die physiologischen Grenzen von 2,3-3 mmol/l gekennzeichnet (Kraft und Dürr 2005). Sie tritt als subklinische Hypokalzämie (Calciummangel) um den Zeitpunkt des Kalbens und in frühen Laktationsphasen auf. Die Gebärparese tritt mit einer Inzidenz von 5% (Horst, 1986), bzw. 6 - 10% (Malz und Meaer, 1992) auf. Sie zählt neben der Ketose zu der häufigsten Stoffwechselstörung (Fürll et al., 1996). Typische Anzeichen einer Gebärparese sind zunächst eine verringerte Futteraufnahme, Unruhe und Nervosität, gefolgt von einem Zustand der allgemeinen Schwäche und Trägheit. Die Magentätigkeit ist herabgesetzt, Kot- und Harnabsatz sistieren. In weiteren Stadien folgt ein Festliegen des Tieres in Brust-Bauch-Lage und komatöse Zustände, die ohne Behandlung zum Tode des Tieres führen. (Blum u. Fischer 1974, Radostits et al. 2000; Allen und Davies 1981; Martig 2002; Oetzel 1988, Horst et al. 1997).

Durch die klinische oder subklinische Hypokalzämie kommt es ferner zur Dystokie, Gebärmuttervorfall, Nachgeburtsverhaltung, Endometritis, Unfruchtbarkeit, Mastitis, Labmagenverlagerung, Ketose und Immunsuppression (Ducusin et al., 2003, Houe et al., 2001, Kimura et al., 2006), denen allesamt therapeutisch begegnet werden muss.

Ohne an eine Theorie gebunden sein zu wollen wird angenommen, dass die erfindungsgemäßen Futterzusätze durch eine verbesserte Verdauung indirekt oder durch Beeinflussung der Ca/Mg-Ionen-Leitfähigkeit der entsprechenden TRP-Kanäle direkt eine erhöhte Calcium- oder Magnesiumionenaufnahme bzw. Mobilisierung derselben bewirken. Weiterhin dürfte eine Korrektur der alkalotischen Stoffwechsellage des Wiederkäuers zu einer Normalisierung der Parathormonausschüttung mit nachfolgender Mobilisation von Calcium aus den Speichern im Knochen führen.

Der Begriff "Linderung" kann neben seiner allgemein anerkannten Bedeutung, das heißt der Linderung der Ursache und/oder der Symptome eines Leidens, auch im Sinne der allgemein anerkannten Bedeutungen des Begriffs "Behandlung", d.h. der Prävention, Verhinderung, Zurückdrängung, Linderung, Besserung, Verlangsamung, des Anhaltens oder die Umkehr der Progression oder Schwere eines pathologischen Zustands oder der Leiden hiervon, hierin benutzt werden. Insbesondere kann hierbei neben der Linderung der Ursache und/oder Symptome einer Gastrointestinaltraktstörung wie der Pansenazidose, auch die Behandlung und Prävention der betreffenden Störung umfasst sein.

Der Begriff "Futtermittelträger" umfasst Futtermittel-Ausgangserzeugnisse oder Wasser, die nach Kombination, Komplexierung oder Aggregation mit einem oder mehreren der TRP-Agonisten und/oder TRP-Antagonisten gemäß der vorliegenden Erfindung den erfindungsgemäßen Futtermittelzusatz oder das Futtermittel darstellt.

In Bezug auf die Benutzung der der TRP-Agonisten und/oder TRP-Antagonisten der vorliegenden Erfindung als Arzneimittel umfasst der Begriff "pharmazeutischer Träger" inerte Inhaltsstoffe, die nach Kombination, Komplexierung oder Aggregation mit einem oder mehreren der TRP-Agonisten und/oder TRP-Antagonisten der vorliegenden Erfindung die erwünschten pharmazeutischen Formulierungen darstellen und die in der Lage sind diese am erwünschten Wirkort (i.e. im Magen) im Tier wieder freizugeben. Demnach umfassen die pharmazeutischen Formulierungen der vorliegenden Erfindung jede Zusammensetzung, die durch Mischen eines Modulators der vorliegenden Erfindung und eines pharmazeutischen Trägers hergestellt wird, die zur Linderung der gastrointestinalen Erkrankungen brauchbar ist, worin die Modulation der TRP-Kanäle nützlich sein kann. Bevorzugte Futtermittelträger oder pharmazeutische Träger bestehen aus oder umfassen Substanzen aus der Gruppe umfassend siliziumhaltige Verbindungen, wie Wollastonit (auch bekannt als Tafelspat, CaSiO3, bzw. genauer Ca₃[Si₃O₉]), gefällten Kieselsäuren, Aerosil, Kieselgur, Aluminiumsilikate, Calciumsilikate, Eisensilikate und Magnesiumsilikate. Neben oder anstelle der siliziumhaltigen Verbindungen können auch solche im Magen--Darm-Trakt der Wiederkäuer oder der (wiederkäuenden) Schwielensohler unlöslichen und ernährungsphysiologisch unbedenklichen Verbindungen wie Calciumsulfat-Dihydrat, Aluminiumoxide und Magnesiumoxide verwendet werden. Bevorzugt werden die voranstehenden Trägersubstanzen in hochdisperser Form mit einer spezifischen Oberfläche von mehr als 20 m²/g verwendet, wodurch ein Entmischen von Trägerstoff und Wirkstoffen (TRP-Modulatoren) verhindert wird. Ohne an eine Theorie gebunden sein zu wollen, wird angenommen, dass die Trägersubstanzen dabei auch als Emulgator fungieren, damit die Futtermittelzusätze der vorliegenden Erfindung zur Freisetzung der Wirkstoffe leichter verdaulich werden. Eine weitere Möglichkeit besteht in der Verabreichung der Substanzen in einer öligen bis fettigen Trägersubstanz. Hierbei können tierische und pflanzliche Fette und fette Öle (Öle) benutzt werden, wobei allerdings pflanzliche Fette und Öle, wie z.B. Kokosfett und Rapsöl bevorzugt werden. In einer Ausführungsform werden bei Benutzung von öligen Trägersubstanzen die festen Bestandteile bevorzugt in dem als Trägerstoff verwendeten Pflanzenöl oder Pflanzenfett dispergiert, bis eine gleichmäßige Verteilung der festen Bestandteile erzielt und durch entsprechende Viskosität ein Entmischen der Wirk- und Trägerstoffe während der Lagerung verhindert wird. Beispiele für das erfindungsgemäß verwendete Pflanzenöl sind Rapsöl, Palmöl, Sojaöl, Olivenöl, Erdnussöl, Sonnenblumenöl und Weizenkeimöl und auch Mischungen dieser Öle, wie z.B. ein Mischöl aus Palmöl und Kokosöl. Bei Benutzung von Pflanzenöl umfasst in einer Ausführungsform der pharmazeutische Träger bzw. die Trägersubstanz aufgrund seiner ernährungsphysiologischen und verarbeitungstechnischen Vorzüge bevorzugt Rapsöl, Palmöl oder Sojaöl oder Mischungen dieser Öle. Beispiele bevorzugter pflanzliche Fette sind Kokosfett und Palmfett, hier insbesondere geschütztes Palmfett, umfassend verseifte Palmölfettsäuren (Ca-Seifen) und Kaltsprühfett sowie auch Mischungen dieser Fette. Daher umfasst in einer Ausführungsform der pharmazeutische Träger bzw. die Trägersubstanz Kokosfett oder Palmfett, bevorzugt geschütztes Palmfett. Ferner umfasst in einer Ausführungsform der pharmazeutische Träger bzw. die Trägersubstanz Mischungen aus mindestens einem der oben benannten Fette mit mindestens einem der oben benannten Öle.

Die Verträglichkeit sowohl des Futtermittelträgers wie des pharmazeutischen Trägers wird dadurch gewährleistet, dass nur Substanzen hierfür gewählt werden, die keinen schädlichen Einfluss bei Einnahme durch das Tier haben.

Der TRP-Agonist bzw. Antagonist kann in einem Pflanzenextrakt umfasst oder davon abgeleitet sein. Vorzugsweise wird der TRP-Agonist und/oder Antagonist als isolierter Wirkstoff bereitgestellt, beispielweise isoliert aus Pflanzenmaterial bzw. Pflanzenextrakt oder hergestellt durch chemische Synthese. Dagegen werden vorzugsweise keine ganzen Pflanzen, die die TRP-Agonisten und/oder Antagonisten der vorliegenden Erfindung beinhalten in den erfindungsgemäßen Futtermittelzusätzen eingesetzt um die hier beschriebenen Wirkungen der TRP-Agonisten und/oder Antagonisten zur Linderung von Gastrointestinaltraktstörungen und damit in Zusammenhang stehender systemischer Störungen in einem Tier aus der Unterordnung der Wiederkäuer oder der (wiederkäuenden) Schwielensohler zu erreichen. Daher liegt in einer bevorzugten Ausführungsform mindestens einer der TRP-Modulatoren als isolierter Wirkstoff in dem Futtermittel der vorliegenden Erfindung vor.

Pellets, Presslinge, Granulat und Kompaktate bezeichnen Formen von Futtermitteln, bzw., Futterzusätzen die nach dem Vermischen ihrer Bestandteile, durch Zusammenpressen im Volumen verkleinert wurden wodurch z.B. Transport- und Lagerkapazitäten eingespart werden können. Da durch das Pressen den Futtermitteln bzw. Futterzusätzen auch Wasser entzogen wird, verlängert sich auch ihre Haltbarkeit. Die Begriffe können zum Teil austauschbar benutzt werden, zum Teil unterscheiden sich diese jedoch in Größe. Unter Presslingen (etwa 2 - 30 mm), Granulaten (etwa 0,5 - 3 mm) und Kompaktaten werden kleinere, rieselfähige Partikel verstanden, Pellets können bis etwa 4 cm groß sein (je Länge oder Durchmesser). Flocken stellen ebenfalls eine gepresste und getrocknete Form dar, die allerdings eher eine zweidimensionale, flache Form aufweist in Vergleich zu den übrigen dreidimensionalen Formen. In einem Ausführungsbeispiel werden die Futtermittelzusätze der vorliegenden Erfindung derart formuliert, dass die Freisetzung der TRP-Modulatoren der vorliegenden Erfindung im Pansen erfolgt. Dies kann z. B. dadurch erreicht werden, dass die isolierten TRP-Modulatoren in eine fetthaltige Trägersubstanz, wie oben definiert eingebettet wird. Durch eine im Pansen vorhandene Temperaturerhöhung (im Gegensatz zur Umgebungstemperatur) kommt es zur Freisetzung der Substanzen. Alternativ kann die Einbettung der isolierten TRP-Modulatoren eine säure-sensible Trägersubstanz erfolgen, wobei eine durch den im Pansen vorhandenen sauren pH-Wert induzierte Freisetzung der TRP-Modulatoren gezielt im Pansen erfolgt.

Der Begriff "Futtermittelzusatz" oder "Futtermittelzusatzstoffe" wird hierin wenn nicht anders angegeben äquivalent benutzt und umfasst Stoffe, Mikroorganismen oder Zubereitungen, die keine Futtermittel-Ausgangserzeugnisse oder Vormischungen sind und bewusst Futtermitteln oder Wasser zugesetzt werden, um eine oder mehrere Funktionen zu erfüllen, wie z.B. die Linderung der hierin definierten Störungen und Krankheiten. Diese Funktionen können alternativ, zusätzlich und teilweise auch adäquat aus der Gruppe gewählt werden umfassend: positive Beeinflussung der Futterbeschaffenheit; positive Beeinflussung der Beschaffenheit der tierischen Erzeugnisse, Deckung des Ernährungsbedarfs der Tiere; positive Beeinflussung der ökologischen Folgen der Tierhaltung; positive Beeinflussung der Tierproduktion, der Leistung oder des Wohlbefindens der Tiere, insbesondere durch Einwirkung auf die Magen- und Darmflora oder die Verdaulichkeit der Futtermittel; Ausüben einer kokzidiostatischen oder histomonostatischen Wirkung.

Der Begriff "Störung" wird weitestgehend äquivalent mit dem Begriff "Erkrankung" oder Krankheit verwendet in dem allgemein bekannten Sinne als bezogen auf einen Zustand von Schwäche, Leiden oder Not eines Säugetieres bedingt durch eine fehlerhafte Funktion eines Organs, der Psyche oder des ganzen Organismus. Ferner wird der Begriff "Störung" zur Beschreibung von ebensolchen Zuständen eines Organismus benutzt, die von der Ausprägung der den Zustand begleitender und dieser kennzeichnender Symptome abgeschwächt sind, während bei einer Erkrankung die Symptome stärker ausgeprägt sind, bis zu für den Organismus lebensbedrohlichen Zuständen. Bezogen auf die medizinische Nomenklatur, wird der Begriff "Störung" auf einen symptomatisch subklinischen bzw. subakuten Schwächezustand eines Organismus bezogen, während der Begriff "Erkrankung", einen klinischen, akuten oder auch chronischen Schwächezustand betrifft.

### Ausführliche Beschreibung der Erfindung

Die vorliegende Erfindung betrifft ein Futtermittelzusatz zur Verwendung in der Linderung von Gastrointestinaltraktstörungen oder damit assoziierter systemischer Störungen durch Modulation eines *Transient Receptor Potential* (TRP)-Kanals in einem Tier aus der Unterordnung der Wiederkäuer (Ruminantia) oder der (wiederkäuenden) Schwielensohler (Tylopoda), enthaltend mindestens einen TRP-Agonisten und/oder Antagonisten. Insbesondere werden erfindungsgemäßen TRP-Agonisten und/oder Antagonisten bzw. diese enthaltende Futtermittelzusätze in der Beeinflussung, d.h. Verbesserung der Resorption von Kationen im Pansen verwendet, vorzugsweise dadurch gekennzeichnet, dass die Kationen Ammonium und/oder Calcium umfassen.

Ansätze zur Optimierung der Stickstoffkonzentration im Pansen für die mikrobielle Fermentation basieren zur Zeit ausschließlich auf einer Anpassung der Zusammensetzung oder der Darreichungsform des Futters oder aber auf Eingriffen in das mikrobielle Fermentationsgeschehen[1-3].

Allerdings spielt für die Stickstoffkonzentration im Pansen neben der Zufuhr mit der Nahrung auch der Umstand eine Rolle, dass große und variable Mengen von Stickstoff in der Form von Ammoniak aus dem Pansen ins Blut gelangen und von dort über die Niere ausgeschieden werden[4-6]. Dieses stellt in zweierlei Hinsicht ein großes Problem in der modernen Nutztierhaltung dar: zum einen muss die Zufuhr von Stickstoff mit der Nahrung (in der Regel in Form von teurem, proteinhaltigem Futter) erhöht werden; zum Anderen treten Probleme bei der Entsorgung der Ausscheidungsprodukte auf, deren hohe Stickstoffgehalte die Umwelt belasten. Wie eine Zusammenstellung aus dem Jahr 2000 durch die Bundeszentrale für politische Bildung zeigt, gehen 95% der NH₃-Emissionen von der Landwirtschaft aus, wobei wiederum 50% davon auf die Haltung von Wiederkäuern zurückzuführen sind [7].

Bisher wurde allgemein davon ausgegangen, dass es sich beim Resorptionsgeschehen, welches zu Verlusten von Stickstoff aus dem Pansen ins Blut führen, um einen ausschließlich passiven Diffusionsvorgang des ungeladenen, lipophilen NH₃-Moleküls durch die Lipidmembran der Pansenepithelzellen handelt. Nicht vereinbar mit dieser Vorstellung sind verschiedene transportphysiologische Beobachtungen zur Resorption von Ammoniak in vivo [8] und in vitro [9].

Ein Ausgangspunkt der vorliegenden Erfindung war die Hypothese, dass neben der Diffusion der lipophilen Form von Ammoniak bei physiologischem pH des Pansens (ca. pH 6.4) große Mengen in der Form von NH₄⁺ über spezielle Proteine ins Blut gelangen können, deren Natur eine pharmakologische Beeinflussung denkbar erscheinen lässt. Dieses konnte im Rahmen der dieser Erfindung zugrundeliegenden Versuche an Organpräparaten (isoliertes, frisches Pansenepithel vom Rind) belegt werden. Hierbei zeigte sich eine durch Ammonium induzierte Leitfähigkeit, welche sich durch erfindungsgemäße Applikation von TRP-Modulatoren wie z.B. Capsaicin und Menthol signifikant beeinflussen ließ. Wie Beispiele 1 und 4 und Figuren 7 bis 10 zeigen, können auch weitere TRP-Modulatoren, wie exemplarisch anhand von Menthol, Thymol, Methylsalicylat und Cinnamaldehyd (auch als Cinnamal oder Zimtaldehyd bezeichnet) gezeigt, zur Beeinflussung der durch Ammonium induzierten Leitfähigkeit im Pansenepithel eingesetzt werden. Die Beeinflussbarkeit der proteinvermittelten Resorptionsmechanismen konnte somit bewiesen werden und stellt die Basis der erfindungsgemäßen Zusammensetzungen dar, die im Nachfolgenden genauer beschrieben werden.

Diese Versuche stützen weiterhin die Arbeitshypothese bezüglich der Natur des modulierten Proteins, weswegen von der Wirkung einer Vielzahl weiterer Substanzen auf die Ammoniumleitfähigkeit des Pansens auszugehen ist (siehe Anhang). Weiterhin konnte gezeigt werden, dass neben der Leitfähigkeit für Ammonium auch die Resorption von Calcium beeinflussbar ist. Es ist davon auszugehen, dass auch die Leitfähigkeit weiterer Kationen (insbesondere Natrium, Kalium, Magnesium, Protonen) durch diese Substanzen beeinflussbar ist, wobei die Futtermittelzusätze der vorliegenden Erfindung den Vorteil bieten, die Resorptionsmechanismen für diese Kationen differenziell regulieren zu können um diese zu optimieren. Mit anderen Worten werden, gemäß der sich durch die erfindungsgemäß durchgeführten Experimente ergebenden Befunde, TRP-Agonisten und/oder Antagonisten vorzugsweise gezielt zur Beeinflussung der Leitfähigkeit von Kationen benutzt, insbesondere von Ammonium- und Calciumionen. Aufgrund dessen eignet sich der erfindungsgemäße Futtermittelzusatz insbesondere zur Behandlung von Krankheiten und Beeinflussung von Bedingungen der Tierhaltung, bei denen der Stoffwechsel von Stickstoff und Calcium eine Rolle spielen und/oder Stoffwechselvorgänge, die von diesen Ionen und deren Resorption im Pansen beeinflusst werden. In einer Ausführungsform ist daher der Futtermittelzusatz der vorliegenden Erfindung, dadurch gekennzeichnet, dass dieser mindestens einen TRP-Agonisten und/oder Antagonisten zur Beeinflussung der Resorption von Kationen im Pansen enthält, wobei die Kationen bevorzugt Ammonium und/oder Calcium umfassen.

Vorzugsweise liegt der mindestens eine TRP-Agonist und/oder Antagonist in einer Konzentration vor, die (umgerechnet) im Wesentlichen derjenigen entspricht, die ausreichend ist die Leitfähigkeit von isolierten Pansenepithel und/oder der epithelialen Pansenzellen für Ammonium gemäß der experimentellen Beschreibung in Beispiel 1 und/oder 2 zu beeinflussen und/oder den Calciumflux über das Pansenepithel gemäß der experimentellen Beschreibung in Beispiel 4.

Vorzugsweise liegt die Konzentration des mindestens einen TRP-Agonisten und/oder Antagonisten max. 10-fach, 9-fach, 8-fach, 7-fach oder 6-fach über dem berechneten Wert, mehr bevorzugt max. 5-fach oder 4-fach, noch mehr bevorzugt max. 3-fach oder 2-fach über dem berechneten Wert und insbesondere bevorzugt max. 1,5-fach über dem berechneten Wert oder bei dem berechneten Wert, vorzugsweise unter Berücksichtigung der empfohlenen Menge der Zugabe an Futtermittelzusatz und Gewicht des zu behandelnden Tieres.

Ein weiterer Vorteil der Zusammensetzungen der vorliegenden Erfindung ergibt sich hieraus, dass eine Beeinflussung der Resorptionsvorgänge am Pansen Auswirkung haben wird auf die Zusammensetzung des Panseninhaltes, wodurch Auswirkungen auf die gesamte Funktion des Pansens (Fermentation, Motorik etc.) und der nachfolgenden Organe (Psalter, Labmagen, Duodenum) zu erwarten sind mit Auswirkungen steigernder Art auf die Tierleistung.

Die Veränderung des I_{sc} durch Zugabe von Menthol und anderer im Anhang genannter TRP-Modulatoren, nachgewiesen in den hier beschriebenen, an Organpräparaten durchgeführten in-vitro-Experimenten, belegt eine Beeinflussung der Ionenleitfähigkeit des Pansenepithels durch diese Stoffe. Ohne sich an eine Theorie binden zu wollen wird anhand von diesen Versuchsergebnissen angenommen, dass die an den Transportvorgängen über das Pansenepithel beteiligten Proteine durch TRP-Modulatoren wie Menthol, als beispielhafter Stellvertreter von Substanzen vom Menthol-Typ, und/oder von Substanzen vom Thymol-Typ, wie z.B. Thymol oder Capsaicin beeinflusst werden können.

Der erfindungsgemäße, vorstehend definierte Futtermittelzusatz ist vorzugsweise dadurch gekennzeichnet, dass der TRP-Kanal zu der Gruppe umfassend TRP Vanniloid 1 (TRPV 1), TRPV2, TRPV3, TRPV4, TRPV5, TRPV6, TRP Ankyrin 1 (TRPA 1), TRP Melastin 3 (TRPM 3), TRPM 5, TRPM 6, TRPM 7 und TRPM 8 gehört.

In einer Ausführungsform ist der erfindungsgemäße Futtermittelzusatz dadurch gekennzeichnet, dass der TRP-Agonist und/oder TRP-Antagonist als isolierter Wirkstoff vorliegt.

Hierbei ist in einer bevorzugten Ausführungsform der vorliegenden Erfindung der Futtermittelzusatz dadurch gekennzeichnet, dass der TRP-Agonist und/oder TRP-Antagonist ausgewählt wird aus der Gruppe bestehend aus Capsaicin (CAS-Nummer 404-86-4), Capsazepin (CAS-Nummer 138977-28-3), Capsiate (CAS-Nummer 205687-01-0), Capsaicinoide, Menthol (CAS-Nummern 2216-51-5, 89-78-1, 15356-60-2, 1490-04-6), Eukalyptol (CAS-Nummer 8024-53-1), Resiniferatoxin (CAS-Nummer 57444-62-9), Resiniferanoide, Piperin (CAS-Nummer 94-62-2), Piperidin (CAS-Nummer 110-89-4), Campher bzw. Kampher (CAS-Nummern 76-22-2, 464-49-3, 464-48-2), bis-Andrographolid (5508-58-7), 2-aminoethyl-dephenylborinat, Icillin (CAS-Nummer 87-08-1), Olvanil (CAS-Nummer 58493-49-5), Verapamil (CAS-Nummer 52-53-9, 152-11-4), Quinidin (CAS-Nummer 6591-63-5), GsMTx4, Johanniskraut, Hyperforin (CAS-Nummer 11079-53-1), Ingwer (Zingiber officinale; CAS-Nummer 84696-15-1), Vanillylaceton (Zingerone; CAS-Nummer 122-48-5), Evadiamin, [6,8,10]Gingerol (CAS-Nummer 23513-14-6), [6,8,10] Shogaol (CAS-Nummer 555-66-8), andere Shogaole, Cannabinol (CAS-Nummer 521-35-7), Cannabidiol (CAS-Nummer 13956-29-1), Cannabis, Tetrahydrocannabinol (THC; CAS-Nummer 1972-08-3), andere Cannabinoide, Polygodial (CAS-Nummer 6754-20-7), Drimanial (CAS-Nummer 352311-05-8), Cinnamodial (CAS-Nummer 23599-45-3), Cinnamosmolide (CAS-Nummer 23599-46-4), Cinnamolide (CAS-Nummer 23599-47-5), Afromodial, Ancistrodial (CAS-Nummer 68398-28-7), Merulidial (CAS-Nummer 68053-32-7), Drimenol (CAS-Nummer 468-68-8), Grifolin (CAS-Nummern 6379-55-1, 6903-07-7), Neogrifolin (CAS-Nummer 23665-96-5), Albaconol, o-Prenylphenol (CAS-Nummer 1128-92-3), BCTC (N-(4-Tertiarybutylphenyl)-4(3-cholorphyridin-2-yl)tetrahydro-pyrazin1(2H)-carboxamid) (CAS-Nummer 393514-24-4), Isovelleral (CAS-Nummer 37841-91-1), Vanillin (CAS-Nummer 121-33-5), Vanillatoxin 1, 2 und 3, Arvanil (CAS-Nummer 128007-31-8), Cnidarian envenomations, Thapsigargin (CAS-Nummer 67526-95-8), Yohimbin (CAS-Nummer 65-19-0), AG489-toxin (CAS-Nummer 128549-96-2), AG505-toxin, Paradol (CAS-Nummer 27113-22-0), Ginsenoside (CAS-Nummer 52286-74-5), Senföl (CAS-Nummer 57-06-7), Allylisothiocyanat (Wasabi; CAS-Nummer 57-06-7), Carvacrol (CAS-Nummer 499-75-2), Carveol (CAS-Nummer 99-48-9), Thymol (CAS-Nummer 89-83-8), Borneol (CAS-Nummern 464-43-7, 464-45-9, 16725-71-6, 10334-13-1, 507-70-0, 124-76-5), Menthon (CAS-Nummern 14073-97-3, 3391-87-5, 10458-14-7), Geraniol (CAS-Nummer 106-24-1), Linalool (CAS-Nummern 78-70-6, 126-91-06, 126-90-9), Menthyllaktat (CAS-Nummer 59259 38 0), p-menthan-3,8-diol (CAS-Nummern 3564-98-5, 3564-95-2, 42822-86-6), L-Carvon (CAS-Nummer 6485-40-1), D-Carvon (CAS-Nummer 2244-16-8), Isopulegol (CAS-Nummer 89-79-2), Hydroxycitronellal (CAS-Nummer 107-75-5), Allicin (CAS-Nummer 539-86-6), Zimtaldehyd (Cinnamaldehyd; CAS-Nummer 104-55-2), Methylsalicilat (CAS-Nummer 119-36-8), Allylisothiocyanat (wasabi) (CAS-Nummer 57-06-7), Benzylisothiocyanat (CAS-Nummer 622-78-6), Phenylethylisothiocyanat (CAS-Nummer 2257-09-2), Isopropylisothiocyanat (CAS-Nummer 2253-73-8), Methylisothiocyanat (CAS-Nummer 556-61-6), Prostanglandine (PGA1 14152-28-4, PGB1 13345-51-2, PGC1 35687-86-6, PGD1 17969-82-0, PGE1 745-65-3, PGE2 363-24-6 , PGF1α 745-62-0, PGG2 51982-36-6 , PGH3 60114-66-1 , PGA2 13345-50-1, PGB2 13367-85-6, PGD3 71902-47-1, PGF2α 551-11-1, PGF2β 4510-16-1, PGC3 52590-97-3, PGI2 35121-78-9), Prostaglandinsyntheseinhibitoren, Acetylsalicylsäure (CAS-Nummer 50-78-2), Paracetamol (CAS-Nummer 103-90-2), lbuprofen (CAS-Nummer 15687-27-1) und Warburganal (CAS-Nummer 62994-47-2). Die CAS-Nummern wurden hier als internationale Identifikationsnummern für die jeweilige Substanz und gegebenenfalls die verschiedener Isoformen in Klammern hinter der Substanz angegeben. Anhand der Nummer können die einzelnen Substanzen und deren Formeln in freien Datenbanken, z.B. unter http://chemsub.online.fr/ oder in Online-Nachschlagewerken wie http://en.wikipedia.org identifiziert und überprüft werden.

Die in den Beispielen, insbesondere in Beispielen 1 und 4, und Figuren 1, 2 und 7 bis 10 dargestellten Ergebnisse belegen, dass durch TRP-Modulatoren erfindungsgemäß die Leitfähigkeit bzw. die Resorption von Kationen über das Pansenepithel moduliert werden kann. Dabei wird zwischen unterschiedlichen Wirkprofilen unterschieden, die zu unterschiedlich nutzbaren Effekten am Gesamtorganismus führen können und aufgrund deren die TRP-Modulatoren der vorliegenden Erfindung wie folgt eingeteilt und bevorzugt eingesetzt werden:

### TRP-Modulatoren, die die Steigerung der Resorption von Kationen (Typ "Menthol") bewirken:

Ohne an eine Theorie gebunden sein zu wollen, wird aufgrund der hier dargestellten Ergebnisse angenommen, dass die Substanzen mit dem Wirkprofil welches hier exemplarisch anhand von Menthol dargestellt wurde (Menthol-Typ-Wirkprofil), besonders geeignet sind, um die Leitfähigkeit des Pansenepithels für Kationen zu beeinflussen und somit z.B. die Resorption von Calcium und/oder Magnesium zu verbessern, die Resorption von Protonen aus dem Pansen zu steigern und/oder die Resorption von Ammonium aus dem Pansen zu steigern. Daher enthalten in einer Ausführungsform die Futtermittelzusätze der vorliegenden Erfindung Substanzen mit dem Menthol-Typ-Wirkprofil, um die Leitfähigkeit des Pansenepithels für Kationen zu modulieren. In einer bevorzugten Ausführungsform enthalten die Futtermittelzusätze der vorliegenden Erfindung Substanzen vom Menthol-Typ-Wirkprofil, um die Resorption von Calcium und/oder Magnesium zu verbessern, die Resorption von Protonen aus dem Pansen zu steigern und/oder die Resorption von Ammonium aus dem Pansen zu steigern.

Die Einordung der Substanzen zu einem der beiden erwähnten Wirkprofiltypen, d.h. zum Menthol-Typ, bzw. zum Thymol-Typ wird in Bezug auf die TRP-Modulatoren der vorliegenden Erfindung bevorzugt anhand deren Eignung zum Erreichen der für die jeweiligen Substanztypen voranstehend und nachfolgend angeführten Effekte auf die Resorption von Ammonium, Calcium, Magnesium, und/oder von Protonen aus dem Pansen vorgenommen. Substanzen, die das gleiche Wirkprofil zeigen, wie in Bezug auf diese Effekte in den Beispielen für Menthol gezeigt, werden dem Menthol-Typ, Substanzen, die das gleiche Wirkprofil zeigen, wie in Bezug auf diese Effekte in den Beispielen für Thymol gezeigt, dem Thymol-Typ zugerechnet.

Dabei werden in einer Ausführungsform als Bestandteil der Futtermittelzusätze der vorliegenden Erfindung als Substanzen mit dem Menthol-Typ-Wirkprofil Substanzen aus der Gruppe umfassend: Menthol, Menthon, Eukalyptol und Geraniol, bevorzugt bestehend aus Menthol, Eukalyptol und Menthon eingesetzt. Besonders bevorzugt wird Menthol als Bestandteil der Futtermittelzusätze der vorliegenden Erfindung als Substanz aus dem Menthol- Wirkprofil Typus eingesetzt.

Aufgrund der Eignung der Substanzen von dem Menthol-Wirkprofiltypus zur Steigerung der Aufnahme von Kationen aus dem Pansen und der daraus folgenden Erhöhung der Blutkonzentration von Magnesium und Calcium und/oder Steigerung der Aufnahme von Ammonium aus dem Pansen und einer daraus folgenden Erhöhung des Pansen-pH ergibt sich ein besonderer Vorteil der Futtermittelzusätze der vorliegenden Erfindung enthaltend die TRP-Modulatoren vom Menthol-Wirkprofil durch deren Eignung für die Prophylaxe und/oder Linderung der Gebärparese/Weidetetanie und/oder die Prophylaxe und/oder Linderung der Pansenazidose. In einem Ausführungsbeispiel werden daher die Futtermittelzusätze der vorliegenden Erfindung enthaltend TRP-Modulatoren vom Menthol-Wirkprofil, besonders bevorzugt Menthol, zur Prophylaxe und/oder Linderung der Gebärparese/Weidetetanie und/oder der Pansenazidose eingesetzt. Ein Nachteil der ausschließlichen Verwendung von TRP-Modulatoren vom Menthol-Typ der vorliegenden Erfindung könnte allerdings in einer Verschlechterung der Stickstoffverwertung durch Verluste von Ammonium aus dem Pansen liegen.

### TRP-Modulatoren, die die Hemmung der Resorption von Kationen (Typ "Thymol") bewirken:

Ohne an eine Theorie gebunden sein zu wollen, wird aufgrund der hier dargestellten Ergebnisse erwartet, dass die Substanzen mit dem Wirkprofil welches hier exemplarisch anhand von Thymol dargestellt wird (Thymol-Typ-Wirkstoffprofil) besonders geeignet sind, um die Resorption von Ammonium aus dem Pansen zu hemmen und/oder die Resorption von Calcium und / oder Magnesium zu verbessern.

Daher werden in einer Ausführungsform als Bestandteil der Futtermittelzusätze der vorliegenden Erfindung Substanzen mit dem Thymol-Typ-Wirkprofil eingesetzt, um die Resorption von Ammonium aus dem Pansen zu hemmen und/oder die Resorption von Calcium und/oder Magnesium zu verbessern. In einer Ausführungsform werden als Substanzen mit dem Thymol-Typ-Wirkprofil Substanzen aus der Gruppe umfassend: Thymol, Cinnamaldehyd (Zimtaldehyd), Methylsalicylat, Carvacrol, Eugenol und Senföl eingesetzt, bevorzugt bestehend aus Thymol, Methylsalicylat, und Zimtaldehyd, besonders bevorzugt bestehend aus Thymol. Bezüglich des Senföls wird ergänzend oder alternativ als ein bevorzugter Bestandteil Allylisothiocyanat eingesetzt.

Anhand der hier beschriebenen Versuchsergebnisse konnte gezeigt werden, dass die beispielhaften TRP-Modulatoren der vorliegenden Erfindung, Thymol, Zimtaldehyd und Methylsalicylat ein ähnliches Wirkprofil am Vormagensystem, speziell dem Pansen aufweisen. Thymol ist ein TRPV3-Agonist. Zimtaldehyd und Methylsalicylat sind beides potente TRPA1-Agonisten, wozu auch Carvacrol, Eugenol und Senföl (dessen eine Wirksubstanz ist Allylisothiocyanat ist) zählen; siehe auch Table 4, auf Seiten 90-91 in Vriens, Nilius et al., Curr Neuropharmacol 6 (2008), 79-96, dessen Offenbarungsgehalt durch Bezugnahme in die vorliegende Anmeldung eingeschlossen ist.

Menthol, ein weiterer beispielhafter TRP-Modulator der vorliegenden Erfindung, zeigte dagegen ein anderes Wirkprofil. Menthol gilt als potenter TRPM8-Agonist, wie auch Menthon, Eucalyptol und Geraniol (siehe Table 3 in Vriens, Nilius, et al. (2008); supra; dessen Offenbarungsgehalt durch Bezugnahme in die vorliegende Anmeldung eingeschlossen ist).

Allerdings kann die Wirkung dieser Substanzen, auch aufgrund deren komplexen Bindungs- und Modulationsverhalten in Bezug auf weitere TRP-Kanäle, an unterschiedlichen Organen auch unterschiedlich sein.

Menthol zählt z. B. ebenfalls zu den TRPV3-Agonisten, allerdings zeigten die mit diesem TRP-Modulator durchgeführten und in den Beispielen beschriebenen Experimente, bei Versuchen am Pansen eine gegenüber Thymol (ebenfalls ein TRPV3-Agonist) entgegengesetzte Wirkung. Dies weist darauf hin, dass eine durch Menthol vermittelte, TRPM8-spezifische Wirkung eintritt, bzw. gegenüber der TRPV3-vermittelten überwiegt.

Eugenol zählt zusätzlich zu seiner Eigenschaft als TRPM8-Agonist auch zu den TRPA1- und TRPV3-Agonisten und wird daher zum Thymol-Typ gezählt, da das hier beschriebene Wirkprofil bereits nachweislich sowohl für TRPA1-(Zimtaldehyd) als auch TRPV3-Agonisten (Thymol) gezeigt wurde.

Die Ergebnisse die in Zusammenhang mit der vorliegenden Erfindung in Bezug auf Menthol, Thymol, Zimtaldehyd und Methylsalicylat hier beschrieben werden sowie die bestehende Literatur können auch genutzt werden um eine Zuordnung der zusätzlich aufgeführten, bevorzugten TRP-Modulatoren der vorliegenden Erfindung zu den beiden vorgenannten Modulator-Typen vorzunehmen.

Ohne an eine Theorie gebunden sein zu wollen wird daher angenommen, dass
- TRP-Modulatoren, die ein Verhalten ähnlich dem hier für Menthol beschriebenen zeigen, d.h. bevorzugt eine TRPM8-agonistische Wirkung haben, dem Menthol-Typ zugerechnet werden können.
- TRP-Modulatoren, die ein Verhalten ähnlich dem hier für Thymol, Zimtaldehyd und Methylsalicylat beschriebenen zeigen, d.h. bevorzugt TRPV3 und/oder TRPA1, allerdings nicht TRPM8 aktivieren, dem Thymol-Typ zugerechnet werden können. Ebenfalls zum Thymol-Typ können demnach auch TRP-Modulatoren gerechnet werden, die TRPV3, TRPA1 und TRPM8 aktivieren, wie z.B. Eugenol.

Ein weiterer besonderer Vorteil der Futtermittelzusätze der vorliegenden Erfindung enthaltend die TRP-Modulatoren vom Thymol-Wirkprofil ergibt sich aus deren Eignung für die Verbesserung der Proteinverwertung durch eine Verminderung der Ammoniumaufnahme vom Pansen ins Blut und einer Erhöhung der Ammoniumkonzentration im Pansen und einen daraus resultierenden vermehrten Einbau von Stickstoff in mikrobielles Protein, woraus folgend auch eine Verminderung der Stickstoffemission in die Umwelt sich ergibt. Ferner zeigen die in den Beispielen dargestellten Ergebnisse die Eignung der TRP-Modulatoren von dem Thymol-Typ zur Prophylaxe und/oder Linderung von Krankheitszuständen geprägt durch Hypokalzämie wie Gebärparese bzw. Hypomagnesiämie wie bei Weidetetanie, aufgrund der durch sie bewirkten erhöhten Resorption von Calcium bzw. von Magnesium. In einem Ausführungsbeispiel werden daher die Futtermittelzusätze der vorliegenden Erfindung enthaltend TRP-Modulatoren vom Thymol-Wirkprofil, besonders bevorzugt Thymol, zur Verbesserung der Proteinverwertung und/oder Prophylaxe und/oder Linderung der Gebärparese/Weidetetanie eingesetzt. Die Futterzusatzmittel der vorliegenden Erfindung enthaltend die hier gekennzeichneten TRP-Modulatoren die erhöhte Calcium-Leitfähigkeit des Pansenepithels bzw. eine erhöhte Calcium-Resorption bewirken, werden zur Prophylaxe bzw. Linderung dieser Krankheit in allen Stadien einer Hypokalzämie eingesetzt. Analog werden die Futterzusatzmittel der vorliegenden Erfindung enthaltend die hier gekennzeichneten TRP-Modulatoren die erhöhte Magnesium-Leitfähigkeit des Pansenepithels bzw. eine erhöhte Magnesium-Resorption bewirken, zur Prophylaxe bzw. Linderung dieser Krankheit in allen Stadien einer Hypomagnesiämie eingesetzt.

### Mischungen der oben erwähnten Substanztypen:

Nach den in den Beispielen gezeigten Ergebnissen könnte mit der Kombination von Substanzen vom Methol/Thymol-Typ, bevorzugt von einer Kombination aus Menthol und Thymol selbst, ein optimales Wirkungsprinzip erzielbar sein mit einer Kombination der Vorteile und weitgehender Vermeidung der Nachteile des Einsatzes der jeweiligen Substanz-Wirkprofil-Typs, wie oben dargelegt also z.B. einer Stimulation der Calcium- und/oder Magnesiumresorption, Prophylaxe und/oder Linderung der Gebärparese bzw. Weidetetanie, und/oder der Pansenazidose und/oder einer Hemmung der Ammoniumresorption / Verbesserung der Stickstoffverwertung und damit einhergehenden weitgehenden Vermeidung der Verschlechterung der Stickstoffverwertung durch Verluste von Ammonium aus dem Pansen.

Als entscheidender Vorteil der Verwendung solcher Kombinationspräparate, d.h. Futtermittelzusätze enthaltend TRP-Modulatoren beider Wirkprofiltypen, also vom Menthol- und Thymol-Typ, erweist sich dabei, dass Thymol offenbar selektiv hemmend auf den Transport einwertiger Kationen (Ammonium) wirkt, ohne mit zweiwertigen Kationen (Ca²⁺ und Mg²⁺) zu interferieren.

In einem Ausführungsbeispiel enthalten hierbei die Futtermittelzusätze der vorliegenden Erfindung vorzugsweise mindestens einen TRP-Modulator aus jeder der beiden Wirkprofiltypen ausgewählt aus der Gruppe umfassend Menthol, Menthon, Thymol, Carvacrol, Geraniol, Zimtaldehyd, Eugenol, Senföl, Citral, und Eukalyptol, besonders bevorzugt aus der Gruppe umfassend Menthol, Menthon, Thymol, Carvacrol, Geraniol, Eugenol und Zimtaldehyd, noch mehr bevorzugt aus der Gruppe umfassend Menthol, Menthon, Thymol, Carvacrol und Eugenol und besonders bevorzugt aus der Gruppe umfassends Menthol und Thymol. In einer bevorzugten Ausführungsform liegt dabei mindestens einer der TRP-Modulatoren als isolierter Wirkstoff in dem Futtermittel der vorliegenden Erfindung vor.

In einer Ausführungsform werden bei der Herstellung der Futtermittelzusätze der vorliegenden Erfindung die TRP-Modulatoren der oben genannten Wirkprofil-Typen zusammengemischt im Mengenverhältnis Menthol-Typ zu Thymol-Typ von 1 : 10 bis 10 : 1; vorzugsweise im Mengenverhältnis Menthol-Typ zu Thymol-Typ von 1 : 3 bis 3 : 1; und besonders bevorzugt im Mengenverhältnis Menthol-Typ zu Thymol-Typ von 1:1 bis 2 : 1.

In einer Ausführungsform bei der Herstellung der Futtermittelzusätze der vorliegenden Erfindung wird dabei eine tägliche Menge bzw. Dosierung der TRP-Modulatoren von dem Menthol-Wirkprofiltyp in der Menge von 10 bis 3000 mg/kg Trockensubstanz (TS) des Kraftfutters (KF) gewählt, bevorzugt 20 bis 500 mg/kg TS KF (ohne Trägersubstanz), stärker bevorzugt 22 bis 333 mg/kg TS KF (ohne Trägersubstanz), noch mehr bevorzugt 22 bis 222 mg/kg TS KF (ohne Trägersubstanz) und besonders bevorzugt 56 bis 166 mg/kg TS KF. In einer bevorzugten Ausführungsform wird hierbei Menthol als bevorzugter Vertreter der Substanzen vom Menthol-Wirkprofiltyp eingesetzt.

In einer Ausführungsform bei der Herstellung der Futtermittelzusätze der vorliegenden Erfindung wird dabei eine tägliche Menge bzw. Dosierung der TRP-Modulatoren von dem Thymol-Wirkprofiltyp in der Menge von 10 bis 3000 mg/kg Trockensubstanz (TS) des Kraftfutters (KF) gewählt, bevorzugt 10 bis 500 mg/kg TS KF, stärker bevorzugt 11 bis 167 mg/kg TS KF, noch mehr bevorzugt 11 bis 111 mg/kg TS KF und besonders bevorzugt 28 bis 84 mg/kg TS KF (jeweils ohne Trägersubstanz). In einer bevorzugten Ausführungsform wird hierbei Thymol als bevorzugter Vertreter der Substanzen vom Thymol-Wirkprofiltyp eingesetzt.

Bei Futtermittelzusätzen der vorliegenden Erfindung, die TRP-Modulatoren beider Wirkprofil-Typen enthalten wird in einer Ausführungsform eine tägliche Gesamtmenge bzw. Dosierung der TRP-Modulatoren beider Wirkprofiltypen von 20 bis 6000 mg/kg Trockensubstanz (TS) des Kraftfutters (KF) gewählt, bevorzugt 20 bis 1000 mg/kg, mehr bevorzugt 20 bis 500 mg/kg TS KF, noch stärker bevorzugt 33 bis 333 mg/kg TS KF und besonders bevorzugt 83 bis 250 mg/kg TS KF (jeweils ohne Trägersubstanz). In einer bevorzugten Ausführungsform werden hierbei Menthol als bevorzugter Vertreter der Substanzen vom Menthol-Wirkprofiltyp und Thymol als bevorzugter Vertreter der Substanzen vom Thymol-Wirkprofiltyp eingesetzt.

In einer Ausführungsform stellt die vorliegende Erfindung daher einen Futtermittelzusatz zur Verfügung, zur Beeinflussung der Resorption von Kationen, wie zum Beispiel Ammonium, Calcium und Magnesium, im Vormagensystem der Wiederkäuer (Ruminantia) oder der (wiederkäuenden) Schwielensohler (Tylopoda), enthaltend mindestens einen *Transient Receptor Potential* (TRP)-Kanal Modulator aus der Gruppe der TRP-Agonisten und/oder Antagonisten, wobei bevorzugt ein verträglicher Futtermittelträger umfasst ist.

Einige der oben aufgeführten Substanzen bzw. solche Substanzen enthaltende Pflanzenextrakte sind bereits in vorangehenden Studien untersucht worden. Die internationale Patentanmeldung WO 2011/153299 A2 beschreibt z.B. die Verfütterung der Oreganopflanze oder Extrakten hieraus zur Beeinflussung des Fermentationsprozesses durch eine Beeinflussung der Verdauungsvorgänge bei Rindern mit dem Ziel einer reduzierten Methanproduktion. Es findet sich allerdings kein Hinweis auf eine Veränderung der Resorptionsmechanismen des Pansenepithels für Ammoniak bzw. Ammonium oder für andere Ionen.

Die Anmeldung US 2009/0004308 A1 beschreibt die Gabe von Benzoesäure als Futterzusatzmittel zusammen mit einer Mischung aus zumindest zwei Substanzen aus der Gruppe bestehend aus Thymol, Eugenol und Piperin, welche in Pflanzenextrakten oder den entsprechenden Pflanzen selbst vorliegen an Geflügel, welches zur besseren Verdauung und schnellerer Gewichtszunahme der Tiere führen soll. Es findet sich dort allerdings keine Offenbarung von Effekten einer solchen Zufütterung auf die Verdauung von Wiederkäuern, die sich im Verdauungsprozess grundsätzlich von Geflügel unterscheiden.

Ando et al. (2003) beschreiben die Zufütterung von Pfefferminze bei Rindern. Sie beobachten ein etwas verbessertes Verdaugeschehen bei den so gefütterten Tieren, allerdings ebenfalls ohne einen Hinweis auf eine Veränderung der Resorptionsmechanismen des Pansenepithels für Ammonium oder für andere Ionen. Ferner beobachten sie auch eine Erniedrigung des Rumen-pH bei den kräuterzugefütterten Tieren, was eine Kontraindikation dieser Zufütterung bei der Pansenazidose-Behandlung darstellen würde. Im Gegensatz zu den dort dargestellten Verfahren und Futtermitteln zielt die vorliegende Erfindung auf ein ungestörtes Fermentationsgeschehen bei gleichzeitiger Beeinflussung der Resorption.

Die gemäß der vorliegenden Erfindung eingesetzten Konzentrationen von TRP-Agonisten und/oder Antagonisten liegen im sehr geringen Dosisbereich. In der Literatur liegen verschiedene *in vitro* und *in vivo* Studien vor, die belegen, dass es bei den gemäß der vorliegenden Erfindung bevorzugten Substanzen und Dosierungen zu keiner Beeinflussung der Mikroorganismenzusammensetzung und der von diesen ausgehenden Fermentation kommt.

So konnten beispielsweise Evans und Martin (Curr Microbiol 41 (2000), 336 -340) bei in vitro Untersuchungen bei Thymol-Mengen von 3-12g/60l keinen Effekt auf Bakterienflora im Pansensaft feststellen. Macheboeuf et al. (Anim. Feed Sci. Technol. 145 (2008) 335-350) konnten bei Einsatz von 1,5mM Thymol (entsprichend 14g/60l Pansensaft) keinen Einfluss auf Methan-, Ammonium-, Acetat- und Propionatproduktion beobachten, die als Indikatoren für mögliche Veränderungen der Bakterienflora benutzt werden konnten. Castillejos et al. (J. Dairy Sci. 89 (2006), 2649-2658) konnten erst bei sehr hohen Konzentrationen ab 500mg/l (entsprechend 30g/60l) signifikanten Einfluss auf pH, NH₃-Konzentration und die Produktion flüchtiger Fettsäuren (VFA - *volatile fatty acids*) erkennen.

Ähnlich liegen in der Literatur auch keine Hinweise darauf vor, dass hohe Dosen Menthol eine Wirkung auf die Mikroorganismenzusammensetzung und die von diesen ausgehende Fermentation hätten. So konnte selbst bei Einsatz von 0.3% Menthol in Bezug auf die Futter-Trockenmasse (entspricht 8g Menthol/Tag) über 30d bei Stieren kein Einfluss auf die Menge an E.coli im Kot der Tiere beobachtet werden (siehe letzter Abschnitt auf Seite 139 der Dissertationsschrift *"Factors influencing Escherichia Coli 0157 colonization of the gastrointestinal track of feedlot cattle"* von Celine C. Aperce an der Kansas State University von 2012). Bei Einsatz von Menthol als Bestandteil in Pfefferminze (Pfefferminzöl) konnten Agarwal et al. (Anim. Feed Sci. Technol. 148 (2009), 321-327) bei 9g/60l Pansensaft zwar beobachten, dass die Methanproduktion gesenkt wurde, ohne allerdings, dass die Produktion flüchtiger Fettsäuren oder die Bakterienzusammensetzung beeinflusst wurden.

Daher ist anhand der hier beschriebenen TRP-Modulatoren und deren Dosierungen davon auszugehen, dass es bei Verfütterung der erfindungsgemäßen Futterzusätze zu keiner Beeinflussung der Mikroorganismenzusammensetzung und der von diesen ausgehenden Fermentation kommt. In einer bevorzugten Ausführungsform beeinflussen daher die erfindungsgemäßen Futterzusätze nicht die Mikroflora im Darm der Wiederkäuer oder Schwielensohler.

Dem gegenüber wurde in den im Zusammenhang mit der vorliegenden Erfindung durchgeführten Experimenten festgestellt, dass das Resorptionsverhalten in Wiederkäuern und (wiederkauenden) Schwielensohlern durch Modulation der TRP-Kanäle mittels TRP-Agonisten und/oder Antagonisten wie beispielsweise Menthol, als Stellvertreter für Substanzen vom Menthol-Typ und/oder von Substanzen vom Thymol-Typ, z. B. Thymol, Capsaicin, Zimtaldehyd oder Verapamil so verändert werden kann, dass es aufgrund des veränderten Ionenflusses zu einer veränderten Resorption von Ammonium und anderer Ionen kommt und so der Entwicklung von Störungen, die auf beispielsweise erhöhter Ionenkonzentration im Gastrointestinaltrakt beruhen entgegengewirkt werden kann.

In einer bevorzugten Ausführungsform liegt der erfindungsgemäße Futtermittelzusatz dadurch gekennzeichnet vor, dass ein verträglicher Futtermittelträger umfasst ist.

Hierbei kann ein beliebiges Futtermittel benutzt werden, das an Wiederkäuer verfüttert wird, z.B. Futtermittel, welche einen Bestandteil aus Gras, Gemüse, Mais oder Silage aus kleinem Korn oder Heu, Kornnebenprodukten, Ölsaaten oder Ölsaatmehlen, Maiskörnern und kleinen Körnern usw. umfassen, um ein ergänzendes Futtermittel zur Verfügung zu stellen.

Die Zugabemenge des erfindungsgemäßen Futtermittelzusatzes kann hierbei pro Kopf pro Tag variieren, in Abhängigkeit von der Verabreichung und der zu fütternden Spezies.

Im Allgemeinen ist der erfindungsgemäße Futtermittelzusatz dadurch gekennzeichnet, dass der TRP-Agonist und/oder TRP-Antagonist in einer Konzentrationsspanne von 0,01 bis 10 g/kg Futter eingesetzt wird.

In Abhängigkeit davon, welche TRP-Kanäle moduliert werden sollen und wie diese Modulierung in Bezug auf die jeweiligen TRP-Kanäle ausfallen soll, kann es von Vorteil sein, statt nur einem, zwei oder mehrere TRP-Modulatoren in einem Futtermittelzusatz zu kombinieren. Der erfindungsgemäße Futtermittelzusatz kann in solchen Fällen 2, 3, 4, 5, 6, 7, 8, 9, 10 oder mehr TRP-Agonisten; 2, 3, 4, 5, 6, 7, 8, 9, 10 oder mehr TRP-Antagonisten; oder 2, 3, 4, 5, 6, 7, 8, 9, 10 oder mehr TRP-Modulatoren, wie in den Tabellen 10 bis 13 aufgelistet, enthalten, von denen einige TRP-Agonisten andere TRP-Antagonisten sind. Einige der Modulatoren weisen unterschiedliche Effekte in Bezug auf unterschiedliche TRP-Kanäle auf. Menthol z.B. ist ein Antagonist des TRPA1-Ionenkanals, allerdings gleichzeitig auch ein Agonist des TRPM8-Ionenkanals. Andere TRP-Modulatoren, wie z.B. Ingwer können mehrere TRP-Kanäle, darunter z.B. TRPV3 beeinflussen (siehe Tabelle 13). In Abhängigkeit von der beabsichtigten Wirkung können 2, 3, 4, 5, 6, 7, 8, 9, 10 oder mehr TRP-Modulatoren, wie in den Tabellen 10 bis 13 aufgeführt, in einem erfindungsgemäßen Futtermittelzusatz enthalten sein. In einer Ausführungsform ist der oben definierte Futtermittelzusatz dadurch gekennzeichnet, dass der Futtermittelzusatz mindestens zwei oder mehr TRP-Agonisten und/oder TRP-Antagonisten enthält. In einer bevorzugten Ausführungsform ist der oben definierte Futtermittelzusatz dadurch gekennzeichnet, dass der Futtermittelzusatz mindestens einen TRP-Modulator vom Menthol-Typ und/oder vom Thymol-Typ enthält, wobei besonders bevorzugt Menthol der TRP-Modulator vom Menthol-Typ ist und Thymol der TRP-Modulator vom Thymol-Typ.

Wie oben dargelegt können die Futtermittelzusätze der vorliegenden Erfindung allgemein zur verbesserten Futterverwertung infolge verbesserten Verdauung, zur Veränderung der hierbei entstehenden Gaskomposition, insbesondere zur Senkung der Ammoniumentstehung sowie auch im Falle von Gastrointestinaltraktstörungen und/oder damit assozierter Störungen/Erkrankungen zur Linderung einer solchen Störung/Krankheit eingesetzt werden.

Daher stellt die vorliegende Erfindung auch ein Futtermittelzusatz wie oben definiert bereit, dadurch gekennzeichnet, dass die Gastrointestinaltraktstörungen oder die damit assoziierte systemische Störungen gelindert werden können, die zur Gruppe umfassend motorische Störungen von Haube und Pansen; Störungen der Vormagenpassage; Hauben- und Pansenentzündung; Hoflund-Syndrom; Fermentationsstörung; subakuter Pansenazidose; subklinischer Pansenazidose; klinischer Pansenazidose; akuter Pansenazidose; Labmagenverlagerung; Klauenrehe; Leberabszessen; Ulzera; Pansenalkalose; faulige Gärung; Pansentympanie; Erkrankungen der Maulhöhle und des Schlundes; Erkrankungen von Psalter, Labmagen, Dünndarm und Dickdarm; Hypomagnesiämie/Weidetetanie; Hypocalcämie/Gebärparese gehören. Insbesondere wird bevorzugt, dass die Gastrointestinaltraktstörung bzw. die damit assoziierte systemische Störung Pansenazidose und Gebärparese umfasst.

TRP-Agonisten und/oder Antagonisten sowie diese enthaltenden erfindungsgemäßen Futterzusätze sind ebenfalls dadurch gekennzeichnet, dass sie neben der Linderung der oben angeführten Gastrointestinaltraktstörungen oder der damit assoziierten systemischen Störungen, dazu geeignet sind, sollte sich z.B. der Zustand des betroffenen Tieres zunächst verschlechtern auch hinübergehend zur Behandlung der entsprechenden Erkrankung eingesetzt zu werden oder auch direkt zur Linderung bei den entsprechenden Erkrankungen eingesetzt werden können, d.h. bei den gleichnamigen Gastrointestinaltraktserkrankungen und/oder den damit assoziierten systemischen Erkrankungen. Die Begriffe Störung und Erkrankung werden in diesem Zusammenhang also äquivalent benutzt in Hinsicht auf die Einsatzmöglichkeit der erfindungsgemäßen TRP-Modulatoren zur deren Linderung.

Die erfindungsgemäßen Futtermittelzusätze sind zunächst für den Einsatz in der komerziellen Tierzucht und/oder Haltung angedacht, sie können nichtdestotrotz sowohl bei domestizierten wie auch bei wilden Tieren eingesetzt werden.

In einer Ausführungsform wird der oben definierte Futtermittelzusatz bereitgestellt, wobei das Tier zu der Gruppe umfassend Rinder, Schafe, Ziegen, Giraffen, Yaks, Rothirsche, Damhirsche, Elche, Rehe, Antilopen, Büffel, Bisons, Trampeltiere, Dromedare, Alpakas, Lamas, Guanakos und Vikunjas gehört.

Die Gabe der erfindungsgemäßen Futtermittelzusätze kann additiv zu den Futtermitteln erfolgen, die den Tieren bereitgestellt werden, z.B. in Form eines Premixes, der dem jeweiligen Futter vor, während oder kurz nach der Verteilung des Futtermittels an die Tiere, diesem beigemischt wird. Da heutzutage die Futtermittel häufig bereits als fertige Mischungen von den Tierhaltern erworben und eingesetzt werden, können die erfindungsgemäßen Futterzusätze ebenfalls schon diesen Fertigmischungen beigefügt sein. Deswegen wird in einer Ausführungsform der Futtermittelzusatz wie oben definiert bereitgestellt, der dadurch gekennzeichnet ist, dass der Futtermittelzusatz in einem Futtermittel eingemischt ist.

Es wird ferner ein Verfahren zur Herstellung eines Futtermittels oder Futterzusatzes offenbart, das die folgenden Schritte umfasst:
(a) Bereitstellung einer Grundzusammensetzung eines Futtermittels oder Futterzusatzes;
(b) Einmischen einer Futtermittelzusatzes und/oder mindestens eines TRP-Agonisten und/oder TRP-Antagonisten wie vorstehend definiert; und
(c) Formulierung des erhaltenen Gemischs als Futtermittel oder Futterzusatz.

Dieses Verfahren ist in einer Ausführungsform dadurch gekennzeichnet, dass die Grundzusammensetzung im Wesentlichen aus einem handelsüblichen Rinderkraftfutter besteht, dem der erfindungsgemäße Futtermittelzusatz beigemengt wird. Eine ungefähre Zusammensetzung eines solchen Futters ist aufgeführt in der Tabelle 9, im Anhang. Diese Zusammensetzung ist nur als ein Beispiel angegeben. Die Futterzusammensetzung kann je nach Einsatzzweck schwanken. Bei Mastfutter, (Kälber)Aufzuchtfutter oder Milchfutter kann z.B. auch Rohfett und/oder Vitamin-/Mineralstoffe-Premixe beigefügt werden und die einzelnen Anteile in der Menge variiert werden (z.B. Rohproteinanteil zwischen etwa 15 und 40%, Rohfaseranteil zwischen etwa 7 und 20%, Rohfettanteil zwischen 2 und 6%, etc.) Konkrete beispielhafte Rinderfutterzusammensetzungen können z.B. der Patentanmeldung DE 102006026512 A1, auf Seite 3, Abschnitt 19 entnommen werden, dort bezeichnet als Erhaltungsfutter oder Mastfutter, deren Offenbarungsgehalt durch Bezugnahme in die vorliegende Anmeldung eingeschlossen ist.

Die TRP-Modulatoren können in unterschiedlicher Form in dem oben beschriebenen, Verfahren eingesetzt werden, umfassend die Pflanzen, bzw. Organismen, in denen sie vorkommen bzw. angereichert werden, Extrakte hieraus, isolierte und/oder synthetisierte Wirkstoffe. In einer Ausführungsform wird das oben definierte Verfahren bereitgestellt, dadurch gekennzeichnet, dass zumindest ein TRP-Agonist und/oder TRP-Antagonist als isolierter Wirkstoff eingemischt wird.

Die vorliegende Erfindung betrifft auch Futtermittel enthaltend einen erfindungsgemäßen Futtermittelzusatz bzw. den mindestens einen der vorstehend genannten TRP-Modulatoren und/oder erhältlich durch das vorstehend beschriebene Verfahren.

Einer der besonderen Vorteile der vorliegenden Erfindung ist, dass es aufgrund der der Erfindung zugrundeliegenden Experimenten möglich ist die Einzelsubstanzen zu definieren, die zur Lösung der oben beschriebenen Aufgabe geeignet sind. Dadurch ist es nicht notwendig, diese Substanzen enthaltende Pflanzenteile an die Tiere zu verfüttern, sondern ist nun möglich, die als aktive Wirkstoffe identifizierten Einzelsubstanzen zu verabreichen und somit den durch diese erzielten Effekt zu verstärken, bzw. erst zutage treten zu lassen durch ihre Isolation von möglicherweise wechselwirkenden Substanzen. In einer Ausführungsform wird daher durch die vorliegende Erfindung das erfindungsgemäße Futtermittel oder der Futterzusatzstoff wie vorstehend definiert bereitgestellt, dadurch gekennzeichnet, dass mindestens ein TRP-Agonist und/oder TRP-Antagonist in erhöhter Konzentration vorliegt, gemessen an dessen Gehalt in rohen Pflanzenmaterial oder einem Pflanzenextrakt.

Das erfindungsgemäße Futtermittel bzw. Futterzusatz kann in unterschiedlichen Formen formuliert werden, die in der Tierfütterung bekannt und gängig sind. In einer Ausführungsform wird das Futtermittel oder Futterzusatz wie vorstehend beschrieben bereitgestellt, dadurch gekennzeichnet, dass das Futtermittel oder der Futterzusatz in der Form umfassend Tablette, Pellet, ummantelte Pellets, Flocken, Granulat, Pressling, Kompaktat, Dragee, Lösung, Pulver, in einem Beutel, Lecksteinbestandteil oder als Tropfen vorliegt.

Ferner wird die Verwendung eines TRP-Agonisten und/oder TRP-Antagonisten wie oben definiert in der Herstellung von Futtermitteln, Futtermittelzusätzen, Futterzusatzstoffen oder Trinkzusätzen offenbart.

Bei akuten oder schweren Gastrointestinaltraktstörungen und/oder den damit assoziierten systemischen Störungen können TRP-Modulatoren erfindungsgemäß auch als ein Arzneimittel eingesetzt werden und, z.B., erst nach Auftreten der Krankheitssymptome verabreicht werden. Deswegen stellt in einer weiteren Ausführungsform die vorliegende Erfindung den TRP-Agonisten und/oder TRP-Antagonisten, wie oben definiert, zur Verwendung als Arzneimittel in der Behandlung von Gastrointestinaltraktstörungen oder den damit assoziierten systemischen Störungen bereit. Auch hier sind die erfindungsgemäßen TRP-Modulatoren dazu geeignet, als Arzneimittel in der Behandlung der entsprechenden Gastrointestinaltraktserkrankungen und/oder den damit assoziierten systemischen Erkrankungen zu dienen.

Schließlich werden die vorstehend beschriebenen und in den Beispielen illustrierten Futtermittelzusätze, Futtermittel und TRP-Modulatoren, d.h. TRP-Agonisten und TRP-Antagonisten zur bzw. in der Modulation der Geschwindigkeit, mit der Kationen aus dem Pansen in einem Tier aus der Unterordnung der Wiederkäuer oder der (wiederkäuenden) Schwielensohler resorbiert werden und/oder zur Erhöhung des Einbaus von Ammoniak in für das Tier verwertbares mikrobielles Protein offenbart.

Diese und andere Ausführungsformen sind offenbart und umfasst durch die Beschreibung und die Beispiele der vorliegenden Erfindung. Weitere Informationen betreffend Materialien, Methoden, Verfahren, Stoffe und Substanzen die gemäß der Lehre der vorliegenden Erfindung eingesetzt werden sollen, können Dokumenten entnommen werden, die in oder mittels öffentlicher Bibliotheken und Datenbanken zugänglich sind. In dieser Hinsicht kann z.B. die Onlinedatenbank "Medline" benutzt werden, die von dem *National Center for Biotechnology Information* und oder der *National Library of Medicine* an dem *National Institutes of Health* bereitgestellt wird. Weitere Datenbanken und Internetadressen, sind dem Durchschnittsfachmann bekannt oder können mit Hilfe von Internetsuchmaschinen ausfindig gemacht werden.

Allgemeine Informationen bezüglich der Symptomatik und Behandlung von Tier und Nutztierkrankheiten kann Standardwerken der Veterinärmedizin entnommen werden, von denen einige beispielhaft hiernach aufgezählt sind: Dirksen, G.; Gründer, H.D.; Stöber, M.; 2002 Innere Medizin und Chirurgie des Rindes. Parey in Blackwell Verlag GmbH, Berlin-Wien; Hartmann, H.; Meyer, H.; 1994 Klinische Pathologie der Haustiere. Gustav Fischer Verlag, Jena, Stuttgart; Hoffmann, W.; 1992 Rinderkrankheiten. Band 1: Innere und chirurgische Erkrankungen, Verlag Eugen Ulmer, Stuttgart, 304-305; Kraft, W.; Dürr, U. M.; 2005 Klinische Labordiagnostik in der Tiermedizin. Schattauer.

Allgemeine Informationen bezüglich der Ernährung von Wiederkäuern und Fütterungsstrategien können auch einer Vielzahl an Standardwerken und Artikeln, die von staatlichen und privaten Institutionen publiziert werden, entnommen werden, von denen einige beispielhaft hiernach aufgezählt sind: Kirchgessner, M.; 2004 Tierernährung. DLG Verlag, Frankfurt am Main; Jeroch, H.; Drochner, W.; Simon, O.; 1999 Ernährung landwirtschaftlicher Nutztiere. Verlag Eugen Ulmer Stuttgart; Hoffmann, M.H.; 1990 Fütterungsregime für Rinder in Tierfütterung, Vol 2. Auflage. Dt. Landwirtschaftsverlag, Berlin, 3485; Piatkowski, B.; Gürtler, H.; Voigt, J.; 1990; Grundzüge der Wiederkäuerernährung. Gustav-Fischer-Verlag, Jena-Stuttgart, 236 p; Steinwidder, A.; 2005 Milchviehfütterung: Tier- und leistungsgerecht. Stocker, 240 p.

Die hierüber befindliche Beschreibung gibt eine allgemeine Offenbarung der vorliegenden Erfindung wieder. Eine Vielzahl an Veröffentlichungen wurde hierbei zitiert. Vollständige Referenzangaben befinden sich am Ende der Beschreibung, unmittelbar vor den Patentansprüchen. Der Offenbarungsgehalt aller zitierten Veröffentlichungen (einschließlich der wissenschaftlichen Literatur, der erteilten Patente, der veröffentlichten Patentanmeldungen, Angaben und Gebrauchsanweisungen der Hersteller, usw.) werden durch Bezugnahme ausdrücklich in die vorliegende Anmeldung eingeschlossen.

Ein detaillierteres Verständnis der vorliegenden Erfindung kann aus den nachfolgenden spezifischen Beispielen gewonnen werden, die allerdings nur zu diesem Zwecke beigefügt und in keiner Weise dazu beabsichtigt sind, den Umfang der Erfindung zu beschränken.

### Beispiele

### Material und Methoden

### Untersuchungsmaterial

### Organpräparate:

- frisch isoliertes Pansenepithel
   - vom Rind (in der Regel aus Schlachthöfen der Umgebung)
   - vom Schaf (Material aus dem FB Veterinärmedizin Berlin)
   - vom Camel (Kollaboration mit Partnern aus Sudan / Saudi Arabien)
- isolierte und kultivierte Zellen aus den hierüber aufgeführten Geweben

### Gewinnung, Präparation und Transport des Epithels

Die Tiere werden mit Bolzenschuß betäubt und durch anschließenden Blutentzug getötet. Der Pansen wird entlang des Schlachtbandes vom Schlachthofpersonal entnommen und zur Gewinnung des Epithels vorgelegt (Rind) oder von den Untersuchern vor Ort entnommen (Schaf).

Die Entnahme des ca. 10cm² großen Pansenepithelstückes erfolgt aus dem ventro-lateralen Bereich (ventraler Pansensack-Vorhof-Übergang) des Pansens. Um grobe Futterbestandteilen zu entfernen, wird das gewonnene Epithel mit reichlich Transportpufferlösung (Zusammensetzung siehe Tabelle 1) gewaschen und anschließend durch "Stripping" von Fett- und Muskelschicht befreit.

Transport und Aufbewahrung von dem Versuch erfolgen in der temperierten (37°C), mit Carbogen (95% O₂ und 5% CO₂) begasten und auf einen pH-Wert von 7,4 eingestellten Transportpufferlösung (Tabelle 1).

### Isolierung der Zellen

Die Isolierung der Zellen erfolgt gemäß etablierter Verfahren (fraktionelle Trypsinierung (Galfi, Neogrady et al. 1981; Schweigel, Lang et al. 1999)). Dazu werden die Zotten des frisch entnommenen Epithels abpräpariert und unter Rühren in Ca²⁺ und Mg²⁺-freier DPBS Puffer Lösung (Dulbecco's PBS + EDTA + 4% Penicillin/Streptomycin (10 000 U·ml-1 / 10 000 µg·ml-1) (Biochrom AG, Berlin, Germany) inkubiert, zu der 0.25 % Trypsin-EDTA (Sigma-Aldrich, St. Louis, Mo., USA) hinzugefügt wird. Nach Inkubation (etwa 45 Minuten) beginnen sich die Zellen der oberen Zelllage (Stratum Corneum) zu lösen. Die Zellsuspension wird durch Filtration (sterile Gaze) vom Gewebematerial getrennt, welches erneut in frischer Lösung inkubiert wird. Diese Prozedur wird so lange wiederholt, bis sich im Überstand die rundlichen Zellen des Stratum basale zeigen, welche durch Zentrifugation vom übrigen Zellmaterial abgetrennt und in kollagenisierten Schälchen (Kollagen A) mit M199 Zellkultur Medium mit 150 ml·l⁻¹ fetalem Kälberserum, L-Glutamin (6.8 mmol·l⁻¹), Nystatin (2.4 ·105 U·l⁻¹), Gentamycin (50 mg·l⁻¹), und Kanamycin (100 mg·l⁻¹) (Biochrom AG, Berlin, Germany or Sigma-Aldrich, St. Louis, Mo., USA) ausgesät wird. Nach Etablierung der Kultur erfolgt ein Wechsel auf DMEM Medium (Pan Biotech GmbH, Eidenbach, Germany; mit 4.5 g·l⁻¹ Glucose, 3.7 g·l⁻¹ NaHCO3, und L-Glutamin) mit 10% fötalem Kälber Serum, HEPES (2%), und Penicillin/Streptomycin (1 %). Nach Erreichen der Konfluenz erfolgt am Versuchstag selbst oder am Tag vor dem Versuch die Aussaat auf Deckgläschen.

### Versuche am intakten Epithel

Die Versuche am intakten Epithel erfolgen wie bereits beschrieben (Abdoun, Stumpff et al. 2005; Abdoun, Stumpff et al. 2010). Bei den verwendeten Messmethoden handelt es sich um eine Kombination aus der von Ussing (1949) entwickelten Ussingkammer- und der Mikroelektrodenmethode.

### Elektrophysiologische Messung mittels der Ussingkammer

Bei der Untersuchung von epithelialen Transportvorgängen mit der Ussingkammer wird das Epithel in eine Spezialkammer eingebaut, welche es ermöglicht, die zwei Seiten des Epithels (apikal: futterseitig; basolateral: blutseitig) getrennt voneinander mit verschieden zusammengesetzten Pufferlösungen zu überspülen, wobei die elektrophysiologischen Parameter (Strom und Spannung) über das Epithel verfolgt werden. Hierdurch gelingt es, zwischen dem Transport einer ungeladenen Substanz (z.B. Ammoniak, NH₃) und dem Transport einer geladenen Substanz (z.B. Ammonium, NH₄⁺) zu unterscheiden, da ersteres ohne Veränderung von Strom und Spannung erfolgt während letzteres mit dem Transfer einer Ladung gekoppelt ist und somit die transepithelialen elektrischen Parameter verändert. Hierbei wird die zeitgleiche Erfassung der elektrophysiologischen Parameter von bis zu 24 verschiedener Epithelstücken des gleichen Tieres durchgeführt, wobei die Versuchslösungen beliebig gewählt werden können.

Unter Zuhilfenahme eines Spezialumbaus wird auch die Reaktion einzelner Epithelien auf sehr rasche und störungsfrei ablaufende Lösungsveränderungen im zeitlichen Verlauf untersucht. Hierdurch lässt sich z.B. sehr gut die Reproduzierbarkeit einer pharmakologischen Intervention am gleichen Epithelstück untersuchen. Diese Modifikation der klassischen Ussingkammertechnik wird bevorzugt, wenn auch nicht ausschließlich, zum vergleichenden "Screening" der verschiedenen Substanzen, die Gegenstand der vorliegenden Erfindung sind, benutzt. Neben dieser Untersuchung, welche Substanzen Wirkung auf Transportprozesse haben, wird die Ussingkammer daher auch eingesetzt, um z.B. die Dosis, in welcher mit messbaren Effekten zu rechnen ist, zu charakterisieren und/oder die transportierte Ionenspezies zu charakterisieren, welche durch die jeweilige Testsubstanz beeinflusst werden.

### Messung des Transportes ("Flux") mittels der Ussingkammer

Ergänzend wird die Transportrate einer Substanz über das Epithel durch Untersuchung der Pufferlösung vor und nach einer definierten Versuchsperiode quantitativ bestimmt (in Zusammenhang mit den Methoden der vorliegenden Erfindung zur Bestimmung des Ammoniakfluxes mittels einer Ammoniakelektrode (Thermo Scientific Orion 9512HPBNWP, Thermo Fisher Scientific Inc., Waltham, MA 02454) bei gleichzeitiger Erfassung von Verschiebungen im Lösungs-pH, oder Messung des Calcium-Fluxes mit radioaktiven Isotopen).

### Messung der intracellulären Parameter mittels Mikroelektrode (apikales Potential und intrazellulärer pH)

Während einzelner Versuche wird eine Mikroelektrode in das Epithel eingeführt, um das Zellmembranpotential und den intrazellulären pH-Wert der Zelle zeitgleich mit den elektrophysiologischen Parametern zu erfassen. Hierdurch ist es möglich, zu unterscheiden ob ein Ion parazellulär (also zwischen den Zellen) oder transzellulär (also durch die Zellen hindurch) transportiert wird. Veränderungen im Membranpotential lassen darauf schließen, dass der Transport transzellulär durch Ionenkanäle erfolgt.

Dieses Verfahren ist experimentell eher zeitaufwendig und wird deswegen zur näheren Charakterisierung von Reaktionen, welche beim Screening der Substanzen in der elektrophysiologischen Messung mittels der Ussingkammer auffallen, eingesetzt.

### Erfassung der transepithelialen elektrophysiologischen Parameter:

Das Epithel wird waagerecht in eine modifizierte Ussingkammer eingespannt, wobei die mucosale Seite nach oben zeigt. Die aus Plexiglas gefertigte Kammer hat eine Querschnittsfläche von 0,79cm², welche auf 1cm² korrigiert wird. Zum Schutz vor Schäden an den Rändern des Epithels *"edge damage"* wird jeweils zwischen Kammer und Epithel ein Silikonring gelegt. Die beiden entstehenden Kammern (mukosal und serosal) werden durch je ein eigenes Perfusionssytem (4- Kanal- Stativ- Pumpe, Ismatec SA) versorgt. Durch Dreiwegehähne ist ein unabhängiger Lösungswechsel in den beiden Kammern zwischen insgesamt acht Lösungen möglich. Während der Aufbewahrung werden die Lösungen in ihren Behältnissen mit 100% O₂ begast. Zur Erwärmung der Lösungen auf 37°C laufen die dünnen Perfusionsschläuche durch eine Wärmeaustauscherpumpe. Die in beiden Kammern vorhandene Absaugvorrichtung (Masterflex R/S, Cole Parmer Instrument Company) garantiert einen schnellen und sicheren Lösungswechsel. Um Lösungswechsel Artefakte bei der Gabe von Agonisten und Antagonisten sicher auszuschließen, werden die Substanzen zu der jeweiligen Lösung zupipettiert, wobei die Begasung für eine rasche Durchmischung sorgt. Druckschwankungen oder eine Unterbrechung des Kammerdurchflusses können so sicher ausgeschlossen werden.

Wie bei der konventionellen Ussingkammermethode sind zur Messung der transepithelialen Potentialdifferenz und des transepithelialen Stroms (I_{SC}) je zwei gewebenahe und gewebeferne Elektroden vorhanden. Die Verbindung zwischen den Ag/AgCl Elektroden, die mit 3 M KCl gefüllt sind, werden über Agarbrücken hergestellt. Wie bei Strecker (Strecker 2011) wird die Meßkammer mit einer "Voltage Clamp"-Anlage (Ussing-Kammer Einheit) in Baueinheit mit einem Mikroelektrodenverstärker (beides Fa. Biomedical Instruments, Germering) verbunden. Ein über der Meßkammer installierter Mikromanipulator (MF 500) bietet die Möglichkeit die Mikroelektrode in feinen µm-Schritten zu bewegen. Zusätzlich befinden sich oberhalb der Aperatur ein Mikroskop sowie eine Kaltlichtquelle.

### Messung mit "double barreled" Mikroelektroden

Die *"double barreled"* Mikroelektrode wird aus zwei Borosilikatkapillaren gefertigt. Ein Kanal dient zur Messung der apikalen Membranpotentialdifferenz und wird mit 0,5 M KCl gefüllt. Der andere Kanal dient zur Messung des intrazellulären pH-Wertes. Dieser Kanal erhält zunächst durch die Silanisierung mit Dichlormethylsilan (Sigma-Aldrich) eine hydrophobe Beschichtung. Nach dem Einfüllen eines flüssigen Ionenaustauscher (Hydrogen-Ionophor I Cocktail A; Fa. Sigma-Aldrich) kommt es zur Bildung einer flüssigen Membran und damit zu einer festen Verbindung des ionenselektiven Ionophors mit der Glaskapillare. Dieses verwendete Ionophor ist selektiv für H⁺ Ionen, was bedeutet, dass ausschließlich diese Ionen über das Ionophor bis in die *"back-fill-solution"* transportiert werden können und zu einer Potentialveränderung führen, die proportional zur vorhandenen H⁺ Konzentration ist. Dieses gemessene Potential muss um den Wert des apikalen Membranpotentials korrigiert werden, was durch Messung mittels der Referenzelektrode ermöglicht wird. Die beiden Kapillaren werden über frisch chlorierte Silberdrähte mit einem hoch-ohmigen Mikroelektrodenverstärker (F-223 A Dual Elektrometer, WPI) verbunden. Dieser misst jeweils das vorhandene Potential über den beiden Kapillaren. Durch die Bildung der Spannungsdifferenz entsteht eine lineare Funktion des pHᵢ (intrazellulärer pH-Wert).

Durch den Einsatz einer Kombination aus *double-barreled* Mikroelektroden und einer Ussingkammer werden bei Bedarf zusätzlich zu transepithelialen Ussingkammermessdaten wie (PDt, I_{sc}, transepitheliale Leitfähigkeit) auch intraepitheliale Messdaten wie PDₐ und pHᵢ erfasst (Strecker 2011).

### Versuchslösungen

Die verwandten Versuchslösungen können im Detail den Tabellen im Anhang entnommen werden.

Menthol (Sigma-Aldrich) wurde in einer Konzentration von 1mol·l⁻¹ in Ethanol gelöst und zu der verwandten ammoniumhaltigen Versuchslösung zupipettiert. Für eine Endkonzentration von 1 mmol·l⁻¹ erfolgte eine Zugabe von 20µl dieser Stammlösung zu 20ml Versuchslösung; für 200µmol·l⁻¹ entsprechend weniger. In Kontrollexperimenten wurden der Versuchslösung entsprechende Mengen von reinem Ethanol zugegeben. Verapamil wurde in DMSO gelöst; Entzug von Calcium und Magnesium erfolgte durch Gabe von EDTA.

### Versuche an isolierten Zellen mittels der Patch-Clamp-Technik

Für die Versuche am intakten Epithel muss zeit- und kostenaufwendig täglich neues Epithel geholt werden. Es wird daher angestrebt, durch Versuche an Zellkulturen ein schnelleres und effizienteres Screening durchzuführen, um diejenigen Substanzen zu identifizieren, bei denen ein Einsatz am intakten Epithel und schließlich am Tier *in vivo* lohnenswert erscheint. Die Patch-Clamp-Technik erlaubt detailliertere Aufschlüsse über Wirkmechanismen der Testsubstanzen und ein stringenter Nachweis, welche Ionenspezies transportiert werden, ist möglich.

Alle Patch-Clamp Experimente werden mit dem standardmäßigen Verfahren durchgeführt werden, welches in Detail in mehreren Publikationen beschrieben wurde. (Stumpff, Strauss et al. 1997; Stumpff, Que et al. 1999; Stumpff, Boxberger et al. 2005; Stumpff, Bondzio et al. 2007; Stumpff, Martens et al. 2009; Stumpff, Georgi et al. 2011), deren Offenbarungsgehalt durch Bezugnahme in die vorliegende Anmeldung eingeschlossen ist.

Dazu werden Borosilicat-Glaskappilaren (Harvard Apparatus, Holliston, MA, USA) mit einem DMZ-Universal-Puller (Zeitz-Instruments, München, Deutschland) zu einer feinen Spitze ausgezogen, die nach Füllung mit der Pipettenlösung (Tabelle 6) mit Hilfe eines Mikromanipulators unter mikroskopischer Kontrolle auf die auf einem Deckgläschen wachsenden Zellen gesetzt wird. Die Zellen werden zunächst mit einer physiologischen Kochsalzlösung überspült (NaCl Lösung, Tabelle 7).

Durch Anlegen eines Unterdruckes gelingt es, eine Fusion der Lipidmembran der Zelle mit der Glaswand der Kapillare zu induzieren ("seal"). Mit einem weiteren ruckartigen Druckpuls gelingt es in einem Teil der Zellen, die zwischen Glaskappilare und Zytosol befindliche Zellmembran so zu rupturieren, dass eine Verbindung zwischen Pipette und Zytosol hergestellt wird. Dabei sollte die Zellmembran weiterhin mit dem Rand der Pipette verschlossen bleiben. Die Pipettenlösung kann jetzt in die Zelle fließen.

Mit Hilfe des Patch-Clamp-Verstärkers (HEKA Elektronic, Lambrecht, Deutschland) gelingt es nun, zwischen einem in der Pipette befindlichen chlorierten Silberdraht und einer im Bad außerhalb der Zelle befindlichen Erdverbindung Spannungspulse anzulegen und die induzierten Ströme über die Zellmembran zu messen. Dabei erfolgt Pulserstellung, Datenaufnahme, Kapazitätsausgleich, Datenfilterung und Datenanalyse automatisch mittels TIDA for Windows (HEKA Elektronic, Lambrecht, Germany). Insbesondere gelingt es so, für jeden Zeitpunkt der Messung eine Strom-Spannungskurve für die untersuchte Zelle zu erstellen, aus welcher man ermitteln kann, welche Ionen in welchem Umfang durch die Zellmembran hindurchgelassen werden.

Die Zufuhr von Lösung in die Perfusionskammer mit den Zellen erfolgt mittels zweier identischer Pumpen (MS/CA4/840, Ismatec, Glattbrugg-Zürich, Switzerland), welche mit Präzisionsschläuchen ausgestattet wurden (Tygon, Ismatec) um identische Flussraten zu gewährleisten (4 ml·min⁻¹). Alle 8 Schläuche werden mittels eines Milli-manifolds (ALA Scientific Instruments, Westbury, NY, USA) in eine Heizkanüle eingeführt, mit der die Temperierung der Lösungen mittels eines Temperaturcontrollers (PH01, multichannel systems, Reutlingen, Germany) auf beliebige Werte ermöglicht wird (in der Regel 37°C). Mittels zwischengeschalteter Dreiwegehähne ermöglicht der Aufbau rasche und störungsfreie Lösungswechsel.

### Beispiel 1: TRP-Modulatoren beeinflussen die Leitfähigkeit von intaktem Pansenepithel für Kationen

Nach Einspannen des Epithels wird dieses zunächst auf beiden Seiten mit dem ammoniumfreien Puffer pH 7.4 (Tabelle 2) überspült. Typischerweise beobachtet man bereits jetzt einen diskreten transepithelialen Strom, entsprechend der Basisaktivität der epithelialen Natriumpumpe (Na⁺/K⁺-ATPase). Gegebenenfalls wird jetzt die Mikroelektrode ins Epithel eingestochen, wenn die gleichzeitige Erfassung intrazellulärer Parameter erforderlich oder erwünscht ist. Bei erfolgreicher Punktion sollte sich ein deutlich negatives Potential (-20mV bis -40mV) einstellen.

Auf der apikalen Seite wird jetzt ammoniumhaltiger Puffer appliziert (10 mM oder 40 mM) pH 6.4 (Tabelle 4 und 5); der Puffer der basolateralen Seite ("Blut") bleibt unverändert. Der sich einstellende pH-Gradient entspricht in etwa den physiologischen Bedingungen im Pansen. Man beobachtet eine Zunahme des transepithelialen Stroms bzw. der transepithelialen Spannung, entsprechend einem Transport des geladenen Ions (sh. Figuren 1 und 2).

Zu dem apikalen Puffer kann nachfolgend die Testsubstanz in gewünschter Menge zugefügt werden. Bei Zugabe des TRP-Kanal-Agonisten Menthol (200µmol·l⁻¹ bis 1 mmol·l⁻¹) zur ammoniumhaltigen Lösung beobachtet man einen weiteren deutlichen Anstieg des transepithelialen Stroms bzw. der transepithelialen Spannung, entsprechend einem vermehrten Transport des Ammonium-Ions (NH₄⁺) durch das Epithel. Bei Zugabe von Verapamil (1 mmol·l⁻¹) kommt es zum Stromabfall. Calciumentzug führt ebenfalls zu einer Stromzunahme. Insgesamt sprechen diese Beobachtungen für einen gemeinsamen Aufnahmeweg für Ammonium und Calcium durch nicht-selektive Kationenkanäle, deren Durchlässigkeit durch verschiedene Substanzen (z.B. Menthol) modulierbar ist (Vriens, Nilius et al. 2008; Nilius and Owsianik 2011).

Die oben dargelegten Versuche in der Ussingkammer wurden weitergeführt und ebenfalls mit weiteren exemplarischen TRP-Agonisten und/oder Antagonisten durchgeführt.

### Wirkung von Ammonium auf den Kurzschlussstrom

In Fig. 7A und untenstehenden Tabelle 14 erkennt man einen durch Zugabe von Ammonium induzierten Strom, welcher nachfolgend durch entsprechende Agonisten moduliert wird.

**Tabelle 14: Statistische Auswertung von Versuchen wie in Fig. 7A; Abkürzungen hier und in nachfolgenden Tabellen: n.s. - nicht signifikant; MW - Mittelwert, SEM - Standardfehler des Mittelwerts (standard error of the mean).**

| **Nr.** | **Lösung** | µA·cm⁻² | | | | | | **normal verteilt** | **p vs 1** | **p vs 3** |
|---|---|---|---|---|---|---|---|---|---|---|
| | | **n** | **MW** | **SEM** | **Median** | **25%** | **75%** | | | |
| 1 | NaCl | 37 | -2,43 | 2,28 | -3,60 | -11,48 | 2,40 | nein | | n.s. |
| 2 | 40mM NH₄⁺ | 37 | -25,73 | 2,14 | -22,32 | -34,23 | -16,76 | | <0.05 | <0.05 |
| 3 | NaCl(w) | 35 | -2,68 | 1,95 | -5,19 | -9,03 | 2,27 | | n.s. | |

### Dosisabhängigkeit der Wirkung von Menthol

Die Wirkung von Menthol auf den Kurzschlussstrom durch das Pansenepithel - entsprechend einem vermehrten Transport von Kationen - wurde bereits oben dargelegt. Im Folgenden werden die Ergebnissse bezüglich der Abhängigkeit der Stromantwort von der Substanzdosis untersucht (sh. Fig. 7B und Tabelle 15).

**Tabelle 15: Dosisabhängigkeit der Mentholwirkung**

| **Nr.** | **Lösung** | µA·cm⁻² | | | | | | **normal verteilt** | **p vs 1** | **p vs 3** |
|---|---|---|---|---|---|---|---|---|---|---|
| | | **n** | **MW** | **SEM** | **Median** | **25%** | **75%** | | | |
| 1 | 40mM NH₄⁺ | 11 | -30,99 | 3,70 | -30,65 | -39.00 | -22,40 | ja | | 0,02 |
| 2 | 200µM Menthol | 11 | -36,34 | 3,66 | -34,21 | -46,11 | -25,74 | | 0,01 | <0.001 |
| 3 | 40mM NH/ (w) | 11 | -26,42 | 2,71 | -25,70 | -35,09 | -19,19 | | 0,02 | |
| 1 | 40mM NH₄⁺ | 12 | -24,20 | 4,51 | -22,76 | -26,99 | -14,71 | ja | | 0,09 |
| 2 | 1mM Menthol | 12 | -28,73 | 4,83 | -26,90 | -34,76 | -18,14 | | 0,02 | 0,00 |
| 3 | 40mM NH₄⁺ (w) | 12 | -20,88 | 3,68 | -19,61 | -27,00 | -16,96 | | 0,09 | |

### Wirkung von Cinnamaldehyd

Mit Zimtaldehyd (Cinnamaldehyd) liegt ein weiterer Wirkstoff vor, welcher den Transport über das Pansenepithel beeinflusst, diesmal mit hemmendem Profil (sh. Fig. 8A und Tabelle 16 hierunten). Die Hemmung des Ammoniumeffluxes aus dem Pansen könnte zu einem vermehrten Einbau von Stickstoff in mikrobielles Protein führen und dadurch die Stickstoffausscheidung in die Umwelt reduzieren. Eine gleichzeitige Hemmung der Calcium und Protonenresorption kann nicht ausgeschlossen werden. Hier sind weitere in vitro Versuche notwendig. Voraussetzung für den Einsatz könnte eine ausreichende Versorgung mit Calcium und eine sparsame Verabreichung von Kraftfutter sein.

**Tabelle 16: Zusammenfassung der Messungen mit Zimtaldehyd**

| **Nr.** | **Lösung** | µA·cm⁻² | | | | | | **normal verteilt** | **p vs 1** | **p vs 3** |
|---|---|---|---|---|---|---|---|---|---|---|
| | | **n** | **MW** | **SEM** | **Median** | **25%** | **75%** | | | |
| 1 | 40mM NH₄⁺ | 7 | -31,53 | 3,15 | -26,52 | -39,66 | -23,39 | ja | (p= 0.313) | |
| 2 | Zimtaldehyd | 7 | -28,39 | 3,26 | -24,03 | -33,10 | -21,07 | | | |
| 3 | 40mM NH₄⁺ (w) | 7 | -28,30 | 4,81 | -27,36 | -35,79 | -23,22 | | | |

### Wirkung von Methylsalicylat

Ein ebenfalls hemmendes Profil mit hemmender Wirkung auf die Stickstoffresorption aus dem Pansen hat Methylsalicylat. Auch hier dürfte bei der Indikation eine Verbesserung der Proteinverwertung bei Verminderung der Stickstoffausscheidung im Vordergrund stehen. Durch die Zugabe kam es im Mittel zu einer maximalen Abnahme des transepithelialen Stroms von -25,44 ± 1,8 µA · cm⁻² auf -21,06 ± 2,97 µA · cm⁻². Bei n=4 war diese Abnahme (noch) nicht signifikant, aber der Trend ist klar.

### Wirkung von Thymol

Bei Thymol fällt die Dauer der erzielten Hemmung auf, welche noch nach dem Auswaschen nachweisbar ist (sh. Fig. 9, und Tabelle 17 hierunter). Diese Substanz dürfte also in besonderem Maße zur Hemmung der Ammoniumresorption einsetzbar sein.

**Tabelle 17: Thymol führ zu einer langfristigen Hemmung der Ammoniumresorption**

| **Nr.** | **Lösung** | µA·cm⁻² | | | | | | **normal verteilt** | **p vs 1** | **p vs 3** |
|---|---|---|---|---|---|---|---|---|---|---|
| | | **n** | **MW** | **SEM** | **Median** | **25%** | **75%** | | | |
| 1 | 40mM NH₄⁺ | 4 | -22,47 | 3,87 | -19,30 | -26,70 | -18,23 | ja | (p = 0.498) | |
| 2 | 100µM Thymol | 4 | -22,19 | 4,00 | -19,35 | -27,37 | -17,01 | | | |
| 3 | 40mM NH₄⁺ (w) | 4 | -15,82 | 9,60 | -20,91 | -29,01 | -2,63 | | | |

### Beispiel 2: TRP-Modulatoren beeinflussen die Leitfähigkeit von isolierten Pansenzellen für Kationen

Zu Beginn des Versuches befinden sich die mit der natriumgluconathaltigen Pipettenlösung (Tabelle 6) gefüllten Zellen in einer NaCl-haltigen Badlösung (Tabelle 7). Die Strom-Spannungskurve zeigt ein auswärtsrektifizierendes Profil mit negativem Umkehrpotential, entsprechend einer hohen Leitfähigkeit der Zelle für Chlorid (= Anionenkanal (Stumpff, Martens et al. 2009)) bei niedriger Basisleitfähigkeit für Natrium (Leonhard-Marek, Stumpff et al. 2005). Wird jetzt die Badperfusion zu einer Lösung umgeschaltet, die als Kation überwiegend Ammonium enthält (Tabelle 8), beobachtet man eine Zunahme des Stromes bei negativem Pipettenpotential, entsprechend einem Influx des Ammonium Ions (NH₄⁺).

In diesem Zusammenhang sei nochmals darauf hingewiesen, dass die Messungen mit der Patch-Clamp-Technik ein schnelleres "Screening" von Kandidaten für die Versuche am intakten Epithel ermöglichen sollen, sie können also additiv als Vorscreening durchgeführt werden, sind aber nicht essentiell für das erfindungsgemäße Screeningverfahren (siehe auch Abschnitt "Versuche an isolierten Zellen mittels der Patch-Clamp-Technik" oben).

### Beispiel 3: Verfütterung von TRP-Modulatoren enthaltenden Futtermitteln an Rinder

Die Kandidatensubstanzen, die in den *in vitro* Versuchen als die vielversprechendsten identifiziert wurden, werden nach randomisierter Verteilung Holstein Rindern, bevorzugt Holstein-Friesian Kühen mit der Standard-Diät verabreicht. Die Rinder-Kontrollgruppe erhält nur die Standard-Diät. Die Datenerhebung erfolgt auf Basis der Evaluierung von Leistungs- und Gesundheitsparametern der Tiere und Daten des smaXtec Bolus (smaXtec animal care sales GmbH; Graz, AUSTRIA)).

Als Leistungsdaten werden z.B. die tägliche/wöchentliche/monatliche oder jährliche (305 Tage) Milchleistung pro Tier (in Kilogramm oder Litern) und die Milchzusammensetzung, z.B. in Bezug auf Fett und Eiweißgehalt routinemäßig erfasst und zwischen den Tieren der beiden Gruppen verglichen. Alternativ oder zusätzlich werden auch die herkömmlichen funktionalen Merkmale wie Exterieur, Fruchtbarkeit, Kalbeverhalten, Totgeburten verglichen.

Als Gesundheitsparameter werden z. B. Befunde (z. B. Erregerart), Lokalisation der Erkrankung (z. B. Striche, Füße etc.) und Behandlungen verzeichnet und verglichen zwischen den beiden Gruppen. Insbesondere werden Krankheiten dabei beobachtet, deren Auftreten und Verlauf verglichen, die potentiell auf eine Pansenzidose zurückzuführen sind, wie z.B. Mastitis, Endometritis und infektiöse Klauenerkrankungen.

Die Erfassung der Gesundheitsparameter kann mittels anerkannter veterinärmedizinischer Methoden stattfinden, wie in der Fachwelt bekannt und beschrieben z.B in "Modern techniques for monitoring high producing dairy cows", Cook et al, In practice 28(2006); 598-603.

Durch den Einsatz des oben erwähnten, speziellen Pansen-Bolus besteht die Möglichkeit automatisch Daten aus dem Pansen im Intervall von 10 Minuten zu erfassen. Zusätzlich werden Blut-, Urin- und Kotproben (unter anderem zur Evaluierung des Calciumspiegels und des Säure-Base-Status) der Tiere genommen und untersucht.

### Beispiel 4: TRP-Modulatoren beeinflussen den Calciumflux über das Pansenepithel

Zum direkten Nachweis einer Modulation des Calciumtransportes über das Pansenepithel wurde in weiteren Versuchen ein radioaktives Calciumisotop (Ca⁴⁵) eingesetzt. Die Messungen wurden in konventionellen Ussingkammern ohne kontinuierliche Perfusion durchgeführt. Hierbei wurde Gewebe von N=4 (Rinder) entnommen und dabei n=85 Epithelien verwendet

Das Isotop wurde auf der mukosalen Seite (entsprechend der Seite, auf welcher sich die Nahrung befindet) eingesetzt und das Auftauchen auf der Blutseite gemessen

### Dosisabhängige Wirkung von Menthol auf den Flux von Calcium über das Pansenepithel

Versuchsansatz: Es wurden drei Gruppen gebildet. Eine Gruppe blieb als Kontrolle unbehandelt. Die beiden anderen Gruppen wurden jeweils mit Menthol in einer Dosis von 200 µM bzw. 1 mM behandelt und mit der Kontrolle (100%) verglichen. Hierbei wurde konzentrationsabhängige Steigerung des Calciumtransports über das Pansengewebe nach Zugabe von Menthol in zwei verschiedenen Konzentrationen im Vergleich zur Kontrollgruppe gemessen.

### Wirkung von Thymol auf den Flux von Calcium über das Pansenepithel

Versuchsansatz: Es wurde zunächst ein Basalwert des Ca-Fluxes über das Pansenepithel ermittelt. Dann wurde eine Substanz zugegeben. Der anschließend gemessene Ca-Flux wurde gemessen und mit dem Ausgangswert (100%) verglichen. In Anbetracht der hemmenden Wirkung von Thymol auf die Resorption von Ammonium aus dem Pansen mit möglicher Verbesserung der Stickstoffverwertung war es von Interesse, zu erfahren, wie dieser Wirkstoff die Calciumresorption beeinflusst (sh Fig. 10B für die Resultate der Messungen). Die bisherigen Ergebnisse deuten aber darauf hin, dass es langfristig zu keiner signifikanten Reduktion des Calciumtransportes kommt, sondern ein Trend zur Steigerung der Calciumresorption sichtbar wird.

Die obigen Ergebnisse zeigen also, dass durch den Einsatz spezifischer TRP-Modulatoren (wie z.B. Menthol) eine Steigerung des Calciumtransports über das Pansenepithel induzierbar ist.

### Anhang: Zusammensetzung der benutzten Versuchslösungen

### - Versuche am intakten Epithel -

**Tabelle 1: Transportpuffer**

| Substanz | mol/l | g/l |
|---|---|---|
| NaCl | 115.00 | 6.72 |
| NaHCO₃ | 25.00 | 2.100 |
| NaH₂PO₄ | 0.40 | 0.06 |
| Na₂HPO₄ | 2.40 | 0.43 |
| KCl | 5.00 | 0.37 |
| HEPES | 10.00 | 2.38 |
| C₆H1₂O₆·H₂O | 5.00 | 0.99 |
| MgCl₂ (1 mol/l) | 1.20 | 0.24 |
| CaCl₂·2H₂O | 1.20 | 0.18 |

| | | |
|---|---|---|
| Begasung: 95% CO₂/5% O₂, ad 300 mOsmol/l (Mannitol); pH 7.4 (Trizma Base) | | |

**Tabelle 2: Ammoniumfreier Puffer pH 7.4**

| Substanz | mol/l | g/l |
|---|---|---|
| NaCl | 70.00 | 4.09 |
| NaH₂PO₄ | 0.40 | 0.06 |
| Na₂HPO₄ | 2.40 | 0.43 |
| KCl | 5.00 | 0.37 |
| C₆H₁₂O₆·H₂O | 5.00 | 0.99 |
| MOPS | 8.00 | 1.67 |
| Glutamin | 2.50 | 0.37 |
| Nagluconat | 30.00 | 6.544 |
| MgCl₂ (1 mol/l) | 1.20 | 0.24 |
| CaCl₂ ·2 H₂O | 1.20 | 0.18 |
| NMDGCl | 40.00 | 7.809 |

| | | |
|---|---|---|
| Begasung: 100% O₂, ad 300 mOsmol/l (Mannitol); pH 7.4 (Trizma Base) | | |

**Tabelle 3: Ammoniumfreier Puffer pH 6.4**

| Substanz | mol/l | g/l |
|---|---|---|
| NaCl | 70.00 | 4.09 |
| NaH₂PO₄ | 0.40 | 0.06 |
| Na₂HPO₄ | 2.40 | 0.43 |
| KCl | 5.00 | 0.37 |
| C₆H₁₂O₆·H₂O | 5.00 | 0.99 |
| MOPS | 8.00 | 1.67 |
| Glutamin | 2.50 | 0.37 |
| Nagluconat | 30.00 | 6.544 |
| MgCl₂ (1 mol/l) | 1.20 | 0.24 |
| CaCl₂ ·2 H₂O | 1.20 | 0.18 |
| NMDGCl | 40.00 | 7.809 |

| | | |
|---|---|---|
| Begasung: 100% O₂, ad 300 mOsmol/l (Mannitol); pH 6.4 (Trizma Base) | | |

**Tabelle 4: Ammoniumhaitiger Puffer pH 6.4 (10 mM)**

| | | |
|---|---|---|
| NaCl | 70.00 | 4.09 |
| NaH₂PO₄ | 0.40 | 0.06 |
| Na₂HPO₄ | 2.40 | 0.43 |
| KCl | 5.00 | 0.37 |
| C₆H₁₂O₆·H₂O | 5.00 | 0.99 |
| MOPS | 8.00 | 1.67 |
| Glutamin | 2.50 | 0.37 |
| Nagluconat | 30.00 | 6.544 |
| MgCl₂ (1 mol/l) | 1.20 | 0.24 |
| CaCl₂·2H₂O | 1.20 | 0.18 |
| NH₄Cl | 10.00 | 0.534 |
| NMDGCl | 30.00 | 5.857 |

| | | |
|---|---|---|
| Begasung: 100% O₂, ad 300 mOsmol/l (Mannitol); pH 6.4 (Trizma Base) | | |

**Tabelle 5: Ammoniumhaltiger Puffer pH 6.4 (40 mM)**

| Substanz | mol/l | g/l |
|---|---|---|
| NaCl | 70.00 | 4.09 |
| NaH₂PO₄ | 0.40 | 0.06 |
| Na₂HPO₄ | 2.40 | 0.43 |
| KCl | 5.00 | 0.37 |
| C₆H₁₂O₆·H₂O | 5.00 | 0.99 |
| MOPS | 8.00 | 1.67 |
| Glutamin | 2.50 | 0.37 |
| Nagluconat | 30.00 | 6.544 |
| MgCl₂ | 1.20 | 0.24 |
| CaCl₂ ·2 H₂O | 1.20 | 0.18 |
| NH₄Cl | 40.00 | 2.138 |

| | | |
|---|---|---|
| Begasung: 100% O₂, ad 300 mOsmol/l (Mannitol); pH 6.4 (Trizma Base) | | |

### - Versuche an isolierten Zellen -

**Tabelle 6: Pipettenlösung**

| Substanz | mol/l | g/l |
|---|---|---|
| KCl | 5.00 | 0.37 |
| NaH₂PO₄ · H₂O | 1.00 | 0.14 |
| Nagluconat | 128.20 | 27.966 |
| Hepes | 10.00 | 2.38 |
| CaCl₂ ·2 H₂O | 2.00 | 0.29 |
| MgCl₂ · 6 H₂O | 1.10 | 0.22 |
| EGTA | 5.00 | 1.90 |
| NaCl | 8.80 | 0.514 |

| | | |
|---|---|---|
| Keine Begasung, ad 290 mosmol/l (mannitol); pH 7.2 (Trizma Base) | | |

*diese Lösung wird gegebenenfalls modifiziert, wenn dieses für die Signaltransdukation erforderlich sein sollte (z.B. Zugabe von MgATP, Reduktion von EGTA, etc.)*

**Tabelle 7: NaCl Lösung**

| Substanz | mol/l | g/l |
|---|---|---|
| KCl | 5.00 | 0.37 |
| NaH₂PO4 · H₂O | 1.00 | 0.14 |
| NaCl | 137.00 | 8.006 |
| Hepes | 10.00 | 2.38 |
| CaCl₂ ·2 H₂O | 1.70 | 0.25 |
| MgCl₂ · 6H₂O | 0.90 | 0.18 |

| | | |
|---|---|---|
| Keine Begasung, ad 290 mOsmol/0l (Mannitol); pH 7.4 (Trizma Base) | | |

**Tabelle 8: NH4Cl Lösung**

| Substanz | mol/l | g/l |
|---|---|---|
| NH₄Cl | 130.00 | 6.95 |
| NaH₂PO₄ · H₂O | 1.00 | 0.14 |
| NaCl | 5.00 | 0.292 |
| HEPES | 10.00 | 2.38 |
| CaCl₂ ·2 H₂O | 1.70 | 0.25 |
| MgCl₂ · 6 H₂O | 0.90 | 0.18 |

| | | |
|---|---|---|
| Keine Begasung, ad 290 mOsmol/l (Mannitol); pH 7.4 (Trizma Base) | | |

### - Futterzusammensetzung -

**Tabelle 9: Beispielhafte Zusammensetzung eines handelsüblichen (Rinderkraft-)Futters**

| Futterkomponente [g/kg Trockensubstanz] | |
|---|---|
| Rohasche | 70 |
| Rohprotein | 200 |
| Rohfaser | 100 |
| Calcium | 9 |
| Phosphat | 7 |
| Magnesium | 4 |
| Kalium | 12 |
| Natrium | 3,5 |
| Chlorid | 5,5 |
| Schwefel | 1,5 |

### - TRP-Kanäle modulierende Substanzen -

**Tabelle 10: TRPV-Modulatoren**

| **TRPV1** | | |
|---|---|---|
| **Substanz** | **Wirkung** | |
| Capsaicin | Agonist | |
| Piperin | Agonist | |
| Eugenol | Agonist | |
| Resiniferatoxin | Agonist | |
| Gingerol | Agonist | |
| Vanillylaceton | Agonist | |
| Evadiamin | Agonist | |
| Cannabidiol | Agonist | |
| Polygodial | Agonist | |
| Isovelleral | Agonist | |
| Camphor | Agonist | |
| Vanillotoxin1,2 and 3 | Agonist | |
| Thapsigargin | Antagonist | |
| Olvanil | Agonist | |
| Capsazepine | Antagonist | |
| Canabidiol | Agonist | |
| | | |

| **TRPV2** | | |
|---|---|---|
| **Substanz** | **Wirkung** | |
| Tetrahydrocanabinol | Agonist | |
| | | |

| **TRPV3** | | |
|---|---|---|
| **Substanz** | **Wirkung** | |
| Carvacrol | Agonist | |
| Eugenol | | unspezifisch |
| Thymol | | unspezifisch |
| Kampher | | unspezifisch |
| Vanillin | | |
| Menthol | | unspezifisch |
| | | |

| **TRPV4** | | |
|---|---|---|
| **Substanz** | **Wirkung** | |
| Bisandrographolid A | Agonist | |

**Tabelle 11.: TRPM-Modulatoren**

| **TRPM8** | | |
|---|---|---|
| **Substanz** | **Wirkung** | |
| Menthol | Agonist | |
| Eucalyptol | Agonist | |
| Menthon | Agonist | |
| Geraniol | Agonist | |
| Linalool | Agonist | |
| Menthyllaktat | Agonist | |
| Cis- and trans-p-menthan-3,8-diol | Agonist | |
| L-Carvon | Agonist | |
| Isopulegol | Agonist | |
| Hydroxyl-citronellal | Agonist | |
| Eugenol | Agonist | aber auch TRPA1 und TRPV1 |
| Zimtaldehyd | Antagonist | Antagonist |
| Ethanol | Antagonist | Inhibiert durch Modulation der PiP2-Interaktion |
| Icilin | Agonist | |
| Menthon | Agonist | |

**Tabelle 12: TRPA-Modulatoren**

| **TRPA1** | | |
|---|---|---|
| **Substanz** | **Wirkung** | |
| Allyl-isothiocyanat | Agonist | |
| Benzyl-isothiocyanat | Agonist | |
| Phenylethyl-isothiocyanat | Agonist | |
| Isopropyl-isothiocyanat | Agonist | |
| Methyl-isothiocyanat | Agonist | |
| Zimtaldehyd | Agonist | |
| Eugenol | Agonist | aber auch TRPM8 und TRPV1 |
| Gingerol | Agonist | |
| Methylsalicilat | Agonist | |
| Allicin | Agonist | aber auchTRPV1 |
| Carvacrol | Agonist | |
| Kampher | Antagonist | |
| Menthol | Antagonist | aber aktiviert TRPM8 |
| Tetrahydrocannabinol | Agonist | |
| Wasabi, Senföl | Agonist | |
| Gelber Senf | Agonist | |

**Tabelle 13: Unspezifische Modulatoren**

| **Unspezifisch wirksam** |
|---|
| (aktivieren eine Reihe von TRP Kanälen, darunter auch den TRPV3) |
| Piperadine |
| Bisandrographolid |
| Icilin |
| Verapamil |
| Quinidin |
| GsMTx4 |
| Hypericum perforatum (Johanniskraut) |
| Hyperforin |
| Capsiate |
| Vanillylaceton (Zingerone) |
| Evodiamine |
| Menthylsalicilat |
| Senföl |
| Ginsenoside |
| Carveol |
| Paradol |
| Resiniferanoide |
| Capsiate |
| andere Capsaicinoide |
| Zingiber officinale |
| Vanillylaceton (Zingerone) |
| Evodiamine |
| [6,8,10]Shogaol |
| andere Shogaole |
| Cannabis |
| Tetrahydrocannabinol |
| Cannabidiol |
| andere Cannabinoide |
| Drimanial |
| Cinnamodial |
| Cinnamosmolide |
| Cinnamolide |
| Afromodial |
| Ancistrodial |
| Merulidial |
| Drimenol |
| Grifolin |
| Neogrifolin |
| Albaconol |
| Prenylphenole |
| BCTC (N(4Tertiarybutylphenyl)4(3cholorphyridin2yl)tetrahydropyrazin 1(2H)carboxamid |
| Isovelleral |
| Vanillotoxin 1, 2 und 3 |
| Arvanil |
| Cnidarian envenomations |
| Thapsigargin |
| Yohimbin |
| AG489 toxin |
| AG505 toxin |
| Paradol |
| Allylisothiocyanat |
| Geraniol |
| Linalool |
| Menthyllactat |
| Cis und trans p menthan3,8 diol |
| L-Carvone |
| Isopulegol |
| Hydroxylcitronellal |
| Borneol |
| Prostanglandine |
| Prostaglandinsyntheseinhibitoren |
| Acetylsalicylsäure |
| Paracetamol |
| Ibuprofen |

### Literatur

[1] De Campeneere S, De Boever JL, Vanacker JM, Messens W, De Brabander DL: Feeding measures to reduce nitrogen excretion in dairy cattle. Arch Anim Nutr 2009, 63(2):87-103.
[2] Vanhatalo A, Kuoppala K, Ahvenjarvi S, Rinne M: Effects of feeding grass or red clever silage cut at two maturity stages in dairy cows. 1. Nitrogen metabolism and supply of amino acids. J Dairy Sci 2009, 92(11):5620-5633.
[3] Remond D, Bernard L, Savary-Auzeloux I, Noziere P: Partitioning of nutrient net fluxes across the portal-drained viscera in sheep fed twice daily: effect of dietary protein degradability. Br J Nutr 2009:1-12.
[4] Abdoun K, Stumpff F, Martens H: Ammonia and urea transport across the rumen epithelium: a review. Anim Health Res Rev 2006, 7(1-2):43-59.
[5] Harmeyer J, Martens H: Aspects of urea metabolism in ruminants with reference to the goat. J Dairy Sci 1980, 63(10):1707-1728.
[6] McDonald IW: The absorption of ammonia from the rumen of the sheep. Biochem J 1948, 42(4):584-587.
[7] http://www.bpb.de/gesellschaft/umwelt/dossier-umwelt/61246/luftverschmutzung
[8] Gärtner K, Decker P, Hili H: Untersuchungen über die Passage von Harnstoff und Ammoniak durch die Pansenwand von Ziegen. Pflugers Arch 1961, 274:281-288.
[9] Abdoun K, Stumpff F, Wolf K, Martens H: Modulation of electroneutral Na transport in sheep rumen epithelium by luminal ammonia. Am J Physiol 2005, 289(3):G508-520.
[10] Stumpff F: Ionic Conductances of the Ruminal Epithelium. Freie Universität Berlin, Habil., Institut für Veterinär-Physiologie; 2010.
   Galfi, P., S. Neogrady, et al. (1981). "Culture of epithelial cells from bovine ruminal mucosa." Vet Res Commun 4(4): 295-300.
   Stumpff, F., O. Strauss, et al. (1997). "Characterization of maxi-K-channels in bovine trabecular meshwork and their activation by cyclic guanosine monophosphate." Invest Ophthalmol Vis Sci 38(9): 1883-1892.
   Schweigel, M., I. Lang, et al. (1999). "Mg(2+) transport in sheep rumen epithelium: evidence for an electrodiffusive uptake mechanism." Am J Physiol 277(5 Pt 1): G976-982.
   Stumpff, F., Y. Que, et al. (1999). "Stimulation of maxi-K channels in trabecular meshwork by tyrosine kinase inhibitors." Invest Ophthalmol Vis Sci 40(7): 1404-1417.
   Abdoun, K., F. Stumpff, et al. (2005). "Modulation of electroneutral Na transport in sheep rumen epithelium by luminal ammonia." Am J Physiol 289(3): G508-520.
   Leonhard-Marek, S., F. Stumpff, et al. (2005). "Basolateral Mg2+/Na+ exchange regulates apical nonselective cation channel in sheep rumen epithelium via cytosolic Mg2+." Am J Physiol 288(4): G630-645.
   Stumpff, F., M. Boxberger, et al. (2005). "Stimulation of cannabinoid (CB1) and prostanoid (EP2) receptors opens BKCa channels and relaxes ocular trabecular meshwork." Exp Eye Res 80(5): 697-708.
   Stumpff, F. and H. Martens (2006). A role for magnesium in the regulation of ruminal sodium transport. Focus on signal transduction research. G. McAlpine. New York, New Nova Science Publishers, Inc. (ISBN 13 978-1-60021-376-2): 37-66.
   Stumpff, F., A. Bondzio, et al. (2007). "Effects of the Bacillus thuringiensis toxin CrylAb on membrane currents of isolated cells of the ruminal epithelium." J Membr Biol 219(1-3): 37-47.
   Vriens, J., B. Nilius, et al. (2008). "Herbal compounds and toxins modulating TRP channels." Curr Neuropharmacol 6(1): 79-96.
   Schweigel, M., J. Kuzinski, et al. (2009). "Rumen epithelial cells adapt magnesium transport to high and low extracellular magnesium conditions." Magnes Res 22(3): 133-150.
   Stumpff, F., H. Martens, et al. (2009). "Cultured ruminal epithelial cells express a largeconductance channel permeable to chloride, bicarbonate, and acetate." Pflugers Arch 457(5): 1003-1022.
   Abdoun, K., F. Stumpff, et al. (2010). "Modulation of urea transport across sheep rumen epithelium in vitro by SCFA and CO2." Am J Physiol 298(2): G190-202.
   Leonhard-Marek, S., F. Stumpff, et al. (2010). "Transport of cations and anions across forestomach epithelia: conclusions from in vitro studies." Animal 4: 1037-1056.
   Nilius, B. and G. Owsianik (2011). "The transient receptor potential family of ion channels." Genome Biol 12(3): 218.
   Strecker, K. (2011). Untersuchungen zur pH-Regulation am Blättermagenepithel des Schafes mit H+-sensitiven Mikroelektroden. FB Veterinärmedizin. Berlin, FU Berlin. 178 Seiten.
   Stumpff, F., M. I. Georgi, et al. (2011). "Sheep rumen and omasum primary cultures and source epithelia: barrier function aligns with expression of tight junction proteins." J Exp Biol 214(Pt 17): 2871-2882.
   Abdoun, K., F. Stumpff, et al. (2005). "Modulation of electroneutral Na transport in sheep rumen epithelium by luminal ammonia." Am J Physiol 289(3): G508-520.
   Allen WM, Davies DC (1981), "Milk fever, hypomagnesaemia and the "downer cow" syndrome." Br. Vet. J. 137, 435-441.
   Blum J W, Fischer JA (1974) "Ätiologie, Pathophysiologie und Prophylaxe der hypocalcämischen Gebärparese des Rindes - eine Übersicht." Schweiz. Arch. Tierheik. 116,603-628.
   Ducusin, R. J., Y. Uzuka, E. Satoh, M. Otani, M. Nishimura, S. Tanabe und T. Sarashina. (2003), "Effects of extracellular Ca2+ on phagocytosis and intracellular Ca2+ concentrations in polymorphonuclear leukocytes of postpartum dairy cows." Res Vet Sci. 75(1):27-32
   Dirksen, G.; Gründer, H.D.; Stöber, M.; (2002), "Innere Medizin und Chirurgie des Rindes." Parey in Blackwell Verlag GmbH, Berlin-Wien.
   Enemark, J.M.; Jorgensen, R.J.; Kristensen, N.B.; (2004) "An evaluation of parameters for the detection of subclinical rumen acidosis in dairy herds." Vet Res Commun 28, 687-709.
   Fürll, M., Jäkel, L., Bauerfeld, J., Groppel, B. (1996): "Gebärpareseprophylaxe mit "Anionenrationen". Prakt. Tierarzt, Coll. Vet. XXVI, 31-34.
   Garrett, E.F.; Pereira, M.N.; Nordlund, K.V.; Armentano, L.E.; Goodger, W.J.; Oetzel, G.R.; (1999) "Diagnostic Methods for the detection of subacute ruminal acidosis in dairy cows." J Dairy Sci 82, 1170-1178
   Gasteiner J, Fallast M, Rosenkranz S, Häusler J, Schneider K, Guggenberger T (2009): "Zum Einsatz einer intraruminalen pH-Datenmesseinheit mit kabelloser Datenübertragung bei Rindern unter verschiedenen Fütterungsbedingungen." Wien Tierärztl Mschrift; Vet. Med. Austria 96 (2009), 188 - 194.
   Gelfert CC, Loeffler LM, Frömer S, Engel M, Männer K, Staufenbiel R. (2010) "Comparison of the impact of different anionic salts on the acid-base status and calcium metabolism in non-lactating, non-pregnant dairy cows." Vet J. Sep;185(3):305-9. Epub 2009 Aug 25.
   Goff, J.P., Horst, R.L., (1993), "Oral administration of calcium salts for treatment of hypocalcemia in cattle." J. Dairy Sci. 76, 101-108.
   Goff, J.P., Brown, T.R., Stokes, S.R., Brawley, C.L., Valdez, F.R., (2002), "Titration of the proper dose of calcium propionate (NutroCAL) to be included in an oral drench for fresh cows." J. Dairy Sci. 85 (Suppl. 1), 189
   Goff JP, Ruiz R, Horst RL. (2004) Relative acidifying activity of anionic salts commonly used to prevent milk fever. J Dairy Sci. May;87(5):1245-55.
   Horst, R.L. (1986), "Regulation of calcium and phosphorus homeostasis in the dairy cow", Journal of Dairy Science 69, 604-616
   Horst, R.L., Goff, J.P. and Reinhardt, T.A., (1994), 'Calcium and vitamin D metabolism in the dairy cow', Journal of Dairy Science 77, pp. 1936-1951
   Horst RL, Goff JP, Reinhardt TA, Buxton DR (1997), "Strategies for preventing milk fever in dairy cattle." J. Dairy Sci. 80, 1269-1280.
   Houe, H., S. Ostergaard, T. Thilsing-Hansen, R. J. Jorgensen, T. Larsen, J. T. Sorensen, J. F. Agger und J. Y. Blom. (2001) "Milk fever and subclinical hypocalcaemia--an evaluation of parameters on incidence risk, diagnosis, risk factors and biological effects as input for a decision support system for disease control." Acta Vet Scand. 42(1):1-29
   Joris Vriens, Bernd Nilius and Rudi Vennekens, (2008), "Herbal Compounds and Toxins Modulating TRP Channels" Current Neuropharmacology 6, 79-96 79
   Kimura, K., T. A. Reinhardt und J. P. Goff. (2006) "Parturition and hypocalcemia blunts calcium signals in immune cells of dairy cattle" J Dairy Sci. 89(7):2588-2595
   Kraft W, Dürr UM (2005) Klinische Labordiagnostik in der Tiermedizin. Schattauer Verlag, Stuttgart.
   Lang, I. and H. Martens (1999). "Na transport in sheep rumen is modulated by voltagedependent cation conductance in apical membrane." Am J Physiol 277(3 Pt 1): G609-618.
   Littledike, E. T., G. W. Engstrom, and M. Sachs. (1986), "Methods for sequential sampling and analysis of renal 25-hydroxyvitamin D3 1-, 24, and 23-hydroxylase activities of dairy cows and calves injected with 1α-hydroxyvitamin D3." J. Dairy Sci. 69:990-997.
   Malz, C., Meyer, C. (1992), "Neue Aspekte zur Pathogenese und Therapie der hypocalcämischen Gebärparese.", Prakt. Tierarzt 73, 507-515.
   Martens, H. and J. Harmeyer (1978). "Magnesium transport by isolated rumen epithelium of sheep." Res Vet Sci 24(2): 161-168.
   Martig J (2002), "Hypokalzämische Gebärlähmung" In: G. Dirksen; H. D. Gründer u. M. Stöber (Hrsg.): "Innere Medizin und Chirurgie des Rindes", 4. Aufl. Blackwell Verlag Berlin, Wien, 1245-1254.
   Nordlund KV and Garrett EF: "Rumenocentesis: a technique for collecting rumen fluid for the diagnosis of subacute rumen acidosis in dairy herds." Bovine Practitioner 1994, 28, 109-112.
   Nordlund KV, Garrett EF, Oetzel GR: "Herd-based rumenocentesis - a clinical approach to the diagnosis of subacute rumen acidosis." Compend. contin. Educ. pract. Vet. 1995, 17, S48-56.
   Oetzel GR (1988), "Parturient paresis and hypocalcemia in ruminant livestock." Vet. Clinics of North America: Food Anim. Pract. 4, 351-364.
   Radostits OM, Gay CG, Blood DC, Hinchcliff KW (2000) Parturient paresis (milk fever).. In: Radostits. O. M., Gay, C. G., Blood, D. C., Hinchcliff, K. W. Veterinary Medicine. A textbook of the diseases of cattle, sheep, pigs, goats and horses. 9th Ed. W.B. Saunders, London, 1420-1435.
   Ramsey, I. S., M. Delling, et al. (2006). "An introduction to TRP channels." Annual Review of Physiology 68: 619-647.
   Schweigel, M., I. Lang, et al. (1999). "Mg(2+) transport in sheep rumen epithelium: evidence for an electrodiffusive uptake mechanism." Am J Physiol 277(5 Pt 1): G976-982.
   Smith, P. L., K. N. Maloney, et al. (2006). "Bisandrographolide from Andrographis paniculata activates TRPV4 channels." Journal of Biological Chemistry 281(40): 29897-29904.
   Leonhard-Marek, S., F. Stumpff, et al. (2005). "Basolateral Mg2+/Na+ exchange regulates apical nonselective cation channel in sheep rumen epithelium via cytosolic Mg2+." Am J Physiol 288(4): G630-645.
   Xu, H., M. Delling, et al. (2006). "Oregano, thyme and clove-derived flavors and skin sensitizers activate specific TRP channels." Nat Neurosci 9(5): 628-635.

## Patentansprüche

1. Futtermittelzusatz zur Verwendung in der Linderung von Gastrointestinaltraktstörungen oder damit assoziierter systemischer Störungen durch Modulation eines *Transient Receptor Potential* (TRP)-Kanals in einem Tier aus der Unterordnung der Wiederkäuer (Ruminantia) oder der (wiederkäuenden) Schwielensohler (Tylopoda), enthaltend mindestens einen TRP-Agonisten und/oder Antagonisten, wobei bevorzugt ein verträglicher Futtermittelträger umfasst ist.

2. Futtermittelzusatz zur Verwendung nach Anspruch 1, **dadurch gekennzeichnet, dass** dieser mindestens einen TRP-Agonisten und/oder Antagonisten zur Beeinflussung der Resorption von Kationen im Pansen enthält.

3. Futtermittelzusatz zur Verwendung nach Anspruch 2, **dadurch gekennzeichnet, dass** die Kationen Ammonium und/oder Calcium umfassen.

4. Futtermittelzusatz zur Verwendung nach einem der Ansprüche 1 bis 3, **dadurch gekennzeichnet, dass** der TRP-Agonist und/oder TRP-Antagonist als isolierter Wirkstoff vorliegt.

5. Futtermittelzusatz zur Verwendung nach einem der Ansprüche 1 bis 4, **dadurch gekennzeichnet, dass** der TRP-Agonist und/oder TRP-Antagonist ausgewählt wird aus der Gruppe umfassend Menthol, Menthon, Eukalyptol, Thymol, Carvacrol, Eugenol, Geraniol, Methylsalicylat und Zimtaldehyd.

6. Futtermittelzusatz Zur Verwendung nach einem der Ansprüche 1 bis 4, **dadurch gekennzeichnet, dass** der TRP-Agonist und/oder TRP-Antagonist Menthol und Thymol umfasst.

7. Futtermittelzusatz zur Verwendung nach einem der Ansprüche 1 bis 6, **dadurch gekennzeichnet, dass** der TRP-Agonist und/oder TRP-Antagonist in einer Konzentrationsspanne von 0,01 bis 10 g/kg Futter eingesetzt wird.

8. Futtermittelzusatz zur Verwendung nach einem der Ansprüche 1 bis 7, **dadurch gekennzeichnet, dass** der Futtermittelzusatz mindestens zwei oder mehr TRP-Agonisten und/oder TRP-Antagonisten enthält.

9. Futtermittelzusatz zur Verwendung nach einem der Ansprüche 1 bis 8, **dadurch gekennzeichnet, dass** die Gastrointestinaltraktstörung bzw. assoziierte systemische Störung Pansenazidose und/oder Gebärparese umfasst.

10. TRP-Agonist und/oder TRP-Antagonist, definiert nach einem der Ansprüche 1 bis 9, zur Verwendung als Arzneimittel in der Behandlung von Gastrointestinaltraktstörungen oder damit assoziierter systemischer Störungen.

## Claims

1. Feed supplement for use in the alleviation of gastrointestinal tract disorders or systemic disorders associated with it by modulation of a Transient Receptor Potential (TRP) channel in an animal from the suborder of the ruminants (Ruminantia) or the (ruminant) tylopods (Tylopoda), containing at least one TRP agonist and/or antagonist, wherein preferably a well tolerable feed carrier is comprised.

2. Feed supplement for use according to Claim 1, **characterized in that** it contains at least one TRP agonist and/or antagonist for influencing the resorption of cations in the rumen.

3. Feed supplement for use according to Claim 2, **characterized in that** the cations comprise ammonia and/or calcium.

4. Feed supplement for use according to any of Claims 1 to 3, **characterized in that** the TRP agonist and/or TRP antagonist is present as an isolated active substance.

5. Feed supplement for use according to any of Claims 1 to 4, **characterized in that** the TRP agonist and/or TRP antagonist is selected from the group comprising menthol, menthone, eucalyptol, thymol, carvacrol, eugenol, geraniol, methyl salicylate and cinnamaldehyde.

6. Feed supplement for use according to any of Claims 1 to 4, **characterized in that** the TRP agonist and/or TRP antagonist comprises menthol and thymol.

7. Feed supplement for use according to any of Claims 1 to 6, **characterized in that** the TRP agonist and/or TRP antagonist is employed in a concentration range of 0.01 to 10 g/kg feed.

8. Feed supplement for use according to any of Claims 1 to 7, **characterized in that** the feed supplement contains at least two or more TRP agonist and/or TRP antagonist.

9. Feed supplement for use according to any of Claims 1 to 8, **characterized in that** the gastrointestinal tract disorder or the associated systemic disorder comprises rumen acidosis and/or parturient paresis.

10. TRP agonist and/or TRP antagonist, defined according to any of Claims 1 to 9, for use as medicine in the treatment of gastrointestinal tract disorders or systemic disorders associated with it.

## Revendications

1. Additif alimentaire pour animaux, pour son utilisation dans l'atténuation de troubles des voies gastro-intestinales ou de troubles systémiques associés à ceux-ci par la modulation d'un canal à potentiel de récepteur transitoire (*Transient Receptor Potential,* TRP) chez un animal du sous-ordre des ruminants (Ruminantia) ou des camélidés (ruminants) (Tylopoda), contenant au moins un agoniste et/ou un antagoniste du TRP, dans lequel est compris de préférence un support d'aliment pour animaux acceptable.

2. Additif alimentaire pour animaux, pour son utilisation selon la revendication 1, **caractérisé en ce que** celui-ci contient au moins un agoniste et/ou un antagoniste du TRP, pour influer sur la résorption de cations dans la panse.

3. Additif alimentaire pour animaux, pour son utilisation selon la revendication 2, **caractérisé en ce que** les cations comprennent l'ammonium et/ou le calcium.

4. Additif alimentaire pour animaux, pour son utilisation selon l'une des revendications 1 à 3, **caractérisé en ce que** l'agoniste du TRP et/ou l'antagoniste du TRP se présente sous forme de substance active isolée.

5. Additif alimentaire pour animaux, pour son utilisation selon l'une des revendications 1 à 4, **caractérisé en ce que** l'agoniste du TRP et/ou l'antagoniste du TRP est choisi dans le groupe comprenant le menthol, la menthone, l'eucalyptol, le thymol, le carvacrol, l'eugénol, le géraniol, le méthylsalicylate et le cinnamaldéhyde.

6. Additif alimentaire pour animaux, pour son utilisation selon l'une des revendications 1 à 4, **caractérisé en ce que** l'agoniste du TRP et/ou l'antagoniste du TRP comprend le menthol et le thymol.

7. Additif alimentaire pour animaux, pour son utilisation selon l'une des revendications 1 à 6, **caractérisé en ce que** l'agoniste du TRP et/ou l'antagoniste du TRP est utilisé dans une plage de concentration de 0,01 à 10 g/kg d'aliment pour animaux.

8. Additif alimentaire pour animaux, pour son utilisation selon l'une des revendications 1 à 7, **caractérisé en ce que** l'additif alimentaire pour animaux contient au moins deux agonistes du TRP et/ou antagonistes du TRP ou plus.

9. Additif alimentaire pour animaux, pour son utilisation selon l'une des revendications 1 à 8, **caractérisé en ce que** le trouble des voies gastro-intestinales ou le trouble systémique associé comprend l'acidose de la panse et/ou la fièvre de lait.

10. Agoniste du TRP et/ou antagoniste du TRP, défini selon l'une des revendications 1 à 9, pour son utilisation comme médicament dans le traitement des troubles des voies gastro-intestinales ou des troubles systémiques associés à ceux-ci.
